# EUROPEAN PATENT APPLICATION

(11) **EP 0 069 373 A1**
(43) Date of publication of application: **12.01.1983**
(21) Application number: 82105984.7
(22) Date of filing: 05.07.1982
(51) Int. Cl.: C07D 499/00, A61K 31/43, C07D 417/12, C07D 205/08, C07D 257/04, C07D 403/12, C07F 7/18, C07F 9/65

(54) **(5R, 6R)-Penem compounds, their production and use**

(30) Priority: 08.07.1981 JP 106758/82
(71) Applicant: Takeda Chemical Industries, Ltd., Chuo-ku, Osaka (JP)
(72) Inventor: Minamida, Isao, Kawabe-gun Hyogo 666-02 (JP); Munata, Mitsuo, Takatsuki Osaka 569 (JP)
(74) Representative: von Kreisler, Alek, Dipl.-Chem.

(57) **Abstract**

A (6R)-substituted-(5R)-penem-3-carboxylic acid derivative having the formula: 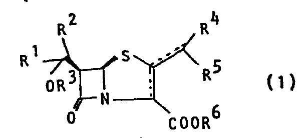 wherein R¹ and R² are the same or different and each is a hydrogen atom or a hydrocarbon group, or R¹ and R² taken together represent an alkylene group; R³ is a hydrogen atom or an acyl group; R⁴ is a hydrogen atom, R⁵ is an alkyl, alkylthio, heterocycle-thio, alkoxy or heterocycle-oxy group which may optionally be substituted, or R⁴ and R⁵ taken together represent a lower alkylene group which may optionally be substituted; and R⁶ is a hydrogen atom or an ester residue, or a pharmacologically acceptable salt thereof and a method of producing the compound (I) by cyclizing a compound of the formula; 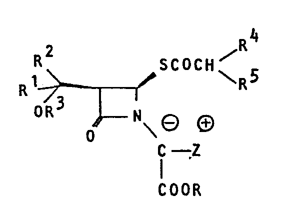 wherein R is an ester residue, Z is a phosphonio or phosphono group and the other groups have the same meanings as defined above, or a salt thereof. And, the compound (I) has strong antibacterial and β-lactamase-inhibiting activities and is of value as drug for animals.

## Description

This invention relates to novel (6R)-substituted-(5R)-penem-3-carboxylic acid derivatives of the formula: 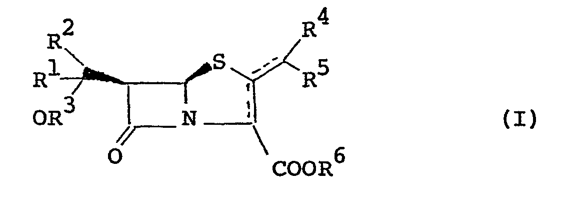 wherein R¹ and R² are the same or different and each is a hydrogen atom or a hydrocarbon group, or R1 and R² taken together represent an alkylene group; R³ is a hydrogen atom or an acyl group; R⁴'is a hydrogen atom, R⁵ is an alkyl, alkylthio, heterocycle-thio, alkoxy or heterocycle-oxy group which may optionally be substituted, or R⁴ and R⁵ taken together represent a lower alkylene group which may optionally be substitued; and R⁶ is a hydrogen atom or an ester residue, or pharmacologically acceptable salts thereof, and a method of producing said derivatives and salts.

While penicillins and cephalosporins still remain to be important antibiotics, the so-called carbapenem compounds such as thienamycin, epithienamycin A to D, MM-13902, MM-17880, MM-4550, C-19393-H₂, C-193935₂, etc. have recently been isolated from the natural kingdom and reported to have strong antibacterial and β-lactamase-inhibiting activities. These compounds have a (5R)-carbapenem ring as the common nucleus and a (substituted) hydroxyalkyl group at 6-position in the S-orientation [trans to (5R)] or in the R-orientation [cis to (5R)]. On the other hand, attempts have been made to synthesize non-natural β-lactam compounds and Woodwards et al synthesized for the first time a penem compound having an acylamino group in (6R)cis-position and reported that the compound has antibacterial activity. Since then, a structural similarity of carbapenem compounds to penem compounds has attracted attention and several 6-(substituted)hydroxyalkylpenem compounds have been synthesized. However, because of the difficulties in the synthetic procedures, majority of the 6-(substituted) hydroxyalkylpenem compounds actually synthesized so far are (6-5 trans)-compounds and the (6-5-cis)-compounds synthesized are limited to (6R)-hydroxymethyl-2-(2'-aminoethylthio)-(5R)-penem-3-carboxylic acid (British Patent No. 2,048,261) and 6-(.l'- hydroxy-l'-ethyl)-2-methylpenem-3-carboxylic acid (British Patent No. 2,042,515). Moreover, the compound disclosed in British Patent No. 2,048,261 can be produced only with a low efficiency and, hence, in a poor yield becuase the antibacterially active, 8-lactamase-inhibiting (5R) compound is obtained after isomerization of the 5- position near by the end of the synthetic process (with a large proportion of the product being the inactive (5S)-compound). The two kinds of compounds disclosed in British Patent No. 2,042,515 are racemic compounds, one of which is a mixture of (1'R, 5R, 6R) compound and (1'S, 5S, 6S) compound which is neither antibacterially active nor S-lactamase-inhibiting, with the other being a mixture of (1'S, 5R, 6R)compound and (1'R, 5S, 6S)compound which is also inactive. Moreover, the production process is complicated and time-consuming because the trans-compound which is produced as a main product in an early stage of the process must be separated from the contemplated cis-compound.

After an intensive research the present inventors found that the optically active (6R-5R-cis)compound (I) can be produced with a high efficiency by cyclizing a compound having the formula: 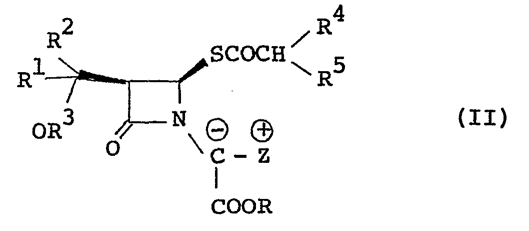 wherein _{R}¹, _{R}², _{R}³, R and R have the meanings defined hereinbefore, R is an ester residue, and Z is a phosphonio or phosphono group and that this particular compound (I) has strong antibacterial and β-lactamase-inhibiting activities. This invention has been accomplished on the basis of the above findings. Therefore, this invention is concerned with:
(1) (6R)-substituted-(5R)-penem-3-carboxylic acid derivatives (I) and
(2) a method of producing a (6R)-substitued-(5R)-penem-3-carboxylic acid derivative of the formula: 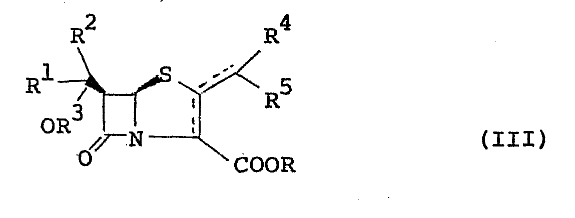 wherein the symbols have the meanings defined hereinbefore, characterized by cyclizing a compound of the formula (II).

Referring to the above formulas, R and _{R}² are the same or different and each is a hydrogen atom or a hydrocarbon group. The hydrocarbon group mentioned just above is typically one of straight-chain or branched lower alkyl groups, e.g. methyl, ethyl, n-propyl, isopropyl, n-butyl, isobutyl, sec-butyl, tert-butyl, etc., aryl groups, e.g. phenyl, tolyl, etc., and aralkyl groups, e.g. benzyl, phenethyl,etc.

Alternatively, R and R² taken together represent an alkylene group of 2 to 7 carbon atoms, such as dimethylene, trimethylene, tetramethylene, -(CH₂)₅- and so on. The symbol R³ means H or an acyl group. As examples of the acyl group may be mentioned lower aliphatic acyl groups such as acetyl, propionyl, n-butyryl, isobutyryl, etc., arylacyl groups such as benzoyl, etc., and aralkylacyl groups such as benz^{y}lcarbonyl, etc. R 4 is H, and R⁵ is an alkyl, alkylthio, heterocycle-thio, alkoxy or heterocycle-oxy group which may optionally be substituted. Examples of said alkyl R⁵ or of the alkyl moiety of said alkylthio R include not only the above-mentioned straight-chain or branched lower alkyl groups but also cycloalkyl groups such as cyclopropyl, cyclobutyl, cyclopentyl, cyclohexyl, etc. The heterocyclic moiety of said heterocycle-thio or heterocycle-oxy group R⁵ may for example a group obtainable on removal of a hydrogen atom from one of the carbon atoms of a 5- to 8-membered ring including one to a few hetero-atoms such as nitrogen (which may be in the oxide form), oxygen or/and sulfur atoms or a fused ring structure comprising such a ring. Thus, for example, there may be mentioned 2- or 3-pyrrolyl, 2- or 3-furyl, 2- or 3-thienyl, 2- or 3-pyrrolidinyl, 2-, 3- or 4-pyridyl, N-oxido-2-, 3-or 4-pyridyl, 2-, 3- or 4-piperidinyl, 2-, 3- or 4-pyranyl, 2-, 3- or 4-thiopyranyl, pyradinyl, 2-, 4- or 5-thiazolyl, 2-, 4- or 5-oxazolyl, 3-, 4- or 5-isothiazolyl, 3-, 4-or 5-isoxazolyl, 2-, 4- or 5-imidazolyl, 3-, 4- or 5-pyrazolyl, 3- or 4-pyridazinyl, N-oxido-3- or 4-pyridazinyl, 2-, 4- or 5-pyrimidinyl, N-oxido-2-, 4- or 5-pyrimidinyl, piperazinyl, 4- or 5-(l,2,3-thiadiazolyl), 3- or 5-(1,2,4-thiadiazolyl), 1,3,4-thiadiazolyl, 1,2,5-thiadiazolyl, 4-or 5-(l,2,3-oxadiazolyl), 3- or 5-(1,2,4-oxadiazolyl), 1,3, 4-oxadiazolyl, 1,2,5-oxadiazolyl, 1,2,3- or 1,2,4-triazolyl, lH- or 2H-tetrazolyl, pyrido[2,3-d]pyrimidyl, benzopyranyl, 1,8-, 1,5-, 1,6-, 1,7-, 2,7- or 2,6-naphthylidyl, quinolyl, thieno[2,3-b]pyridyl, etc. The alkoxy group R⁵ includes straight-chain or branched lower (C₁₋₆) alkoxy groups such as methoxy, ethoxy, n-propoxy, isopropoxy, n-butoxy, iso- butoxy, sec-butoxy, tert-butoxy, n-pentoxy, isopentoxy, etc. The substituents which may be present on the alkyl, alkylthio, heterocycle-thio, alkoxy and heterocycle-oxy groups R⁵ include, among others, alkyl, alkoxy, aryl, aralkyl, mercapto, alkylthio, arylthio, aralkylthio, alkylsulfonyl, arylsulfonyl, aralkylsulfonyl, trihaloalkyl, hydroxy, oxo, thioxo, halo, nitro, amino, cyano, carbamoyl, carboxy, acyl, acyloxy, acylamino, hydroxyalkyl, carboxyalkyl, haloalkyl, mono- or dialkylaminoalkyl, etc. [The alkyl, alkoxy, aryl, aralkyl and acyl groups or moieties are such as those mentioned hereinbefore]. Specific examples of R⁵ are lower alkyl groups (e.g. methyl, ethyl, propyl, butyl, etc.), cycloalkyl groups or lower alkylthio groups (e.g. methylthio, ethylthio, etc.), which may optionally be substituted by amino, dimethylamino, acetylamino, formimidoyl, acetimidoyl, hydroxy, methoxy, ethoxy, cetyloxy, carboxy, methoxycarbonyl, ethoxycarbonyl, methylthio, ethylthio or the like; heterocycle-thio groups such as (1-methyl-lH-tetrazol-5-yl)thio, (5-methyl-l,3,4-thiadiazol-2-yl)thio, (l,3,4-thiadiazol-2-yl)thio, (1-methyl-1,3,4-triazol-2-yl)thio, (1,5-dimethyl-1,3,4-triazol-2-yl)thio, (4-methylthiazol-2-yl)thio, etc.; lower alkyloxy groups such as methoxy, ethoxy, etc.; and heterocycle-oxy groups such as (l-methyl-lH-tetrazol-5-yl)oxy, (5-methyl-l,3,4-thiadiazol-2-yl)oxy, (l,3,4-thiadiazol-2-yl)oxy, (1-methyl-1,3,4-triazol-2-yl)oxy, (1,5-dimethyl-1,3,4-triazol-2-yl)oxy, (4-methylthiazol-2-yl)oxy, etc. R⁴ and R⁵ may, taken together, represent a lower alkylene group which may optionally be substituted. This lower alkylene group may be one corresponding to R¹ and R² taken together, and the substituents which may be present thereon may for example be one of the substituent groups mentioned for R⁵. The ester residues R⁶ and R may for example be straight-chain or branched chain lower alkyl groups, e.g. methyl, ethyl, n-propyl, isopropyl, n-butyl, isobutyl, tert-butyl, etc.; halo-lower alkyl groups, e.g. 2-iodoethyl, 2,2-dibromoethyl, 2,2,2-trichloroethyl, etc.; lower alkoxymethyl groups such as methoxymethyl, ethoxymethyl, n-propoxymethyl, isopropoxymethyl, n-butoxymethyl, isobutoxymethyl, etc.; lower aliphatic acyloxymethyl groups, e.g. acetoxymethyl, propionyloxymethyl, n-butyryloxymethyl, isobutyryloxymethyl, pivaloyloxymethyl, etc.; 1- lower alkoxycarbonyloxyethyl groups, e.g. 1-methoxycarbonyloxy- ethyl, 1-ethoxycarbonyloxyethyl, 1-n-propoxycarbonyloxyethyl, 1-isopropoxycarbonyloxyethyl, 1-n-butoxycarbonyloxyethyl, l-isobutoxycarbonyloxyethyl, etc.; aralkyl groups, e.g. benzyl, p-methoxybenzyl, o-nitrobenzyl, p-nitrobenzyl, etc.; benzhydryl; phthalidyl; etc. Moreover, 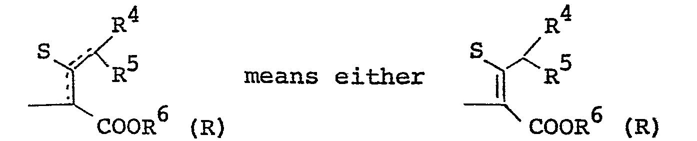 or 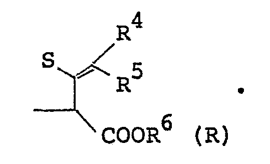 and in the latter meaning, further R⁵ means (E) or (Z) configuration to sulfur atom.

Particularly preferred are cases in which _{R}¹ and _{R}² are the same or different and each is a hydrogen atom or an alkyl group of 1 to 4 carbon atoms, e.g. methyl or ethyl; R¹ and R² taken together represent a lower alkylene group of 3 to 6 carbon atoms such as trimethylene or tetramethylene; R³ is a hydrogen atom or an aliphatic acyl group of 2 to 5 carbon atoms, e.g. acetyl or propionyl; R⁴ is a - hydrogen atom and R⁵ is a heterocycle-thio group, e.g. (l-methyl-lH-tetrazol-5-yl)thio, (1-carboxymethyl-lH- tetrazol-5-yl)thio, [1-(2-dimethylaminoethyl)-1H-tetrazol-5-yl)]thio, (5-methyl-l,3,4-thiadiazol-2-yl)thio or (1-methyl-l,3,4-triazol-2-yl)thio, a substituted lower alkylthio group, e.g. 2-aminoethylthio, 2-formimidoylaminoethyl- thio or 2-acetylaminoethylthio, or a substituted lower alkyl group, e.g. 3-aminopropyl, 3-formimidoylpropyl or 3- acetylaminopropyl; or R⁴ and R⁵ taken together represent a substituted lower alkylene group, e.g. 2-aminopropylene, 2-formimidoylaminopropylene or 2-acetylaminopropylene; and R⁶ is a hydrogen atom, pivaloyloxymethyl or acetoxymethyl.

The following is a partial listing of specific (6R)-substituted-(5R)-penem-3-carboxylic acid derivatives of the formula (I) according to this invention.

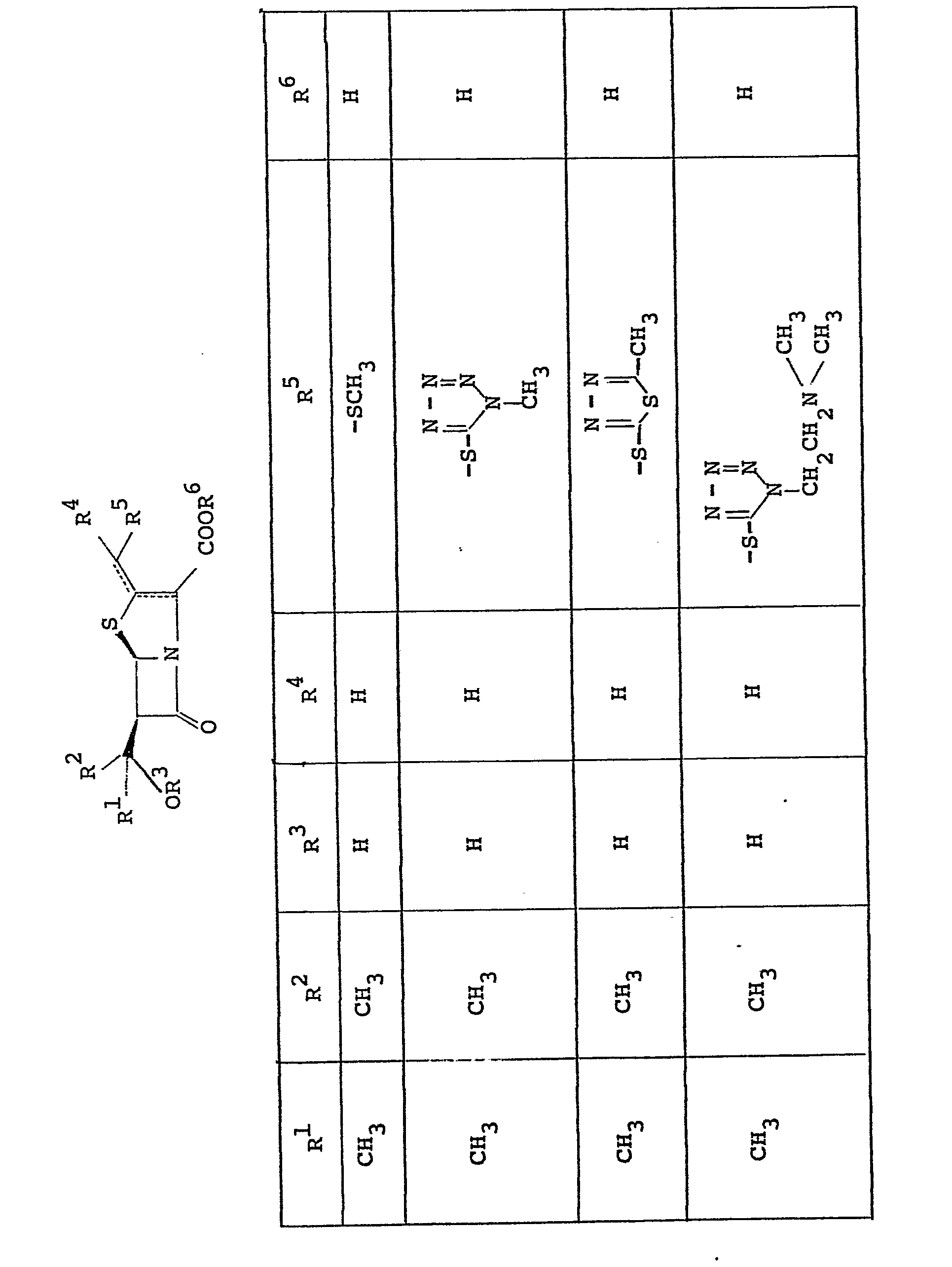

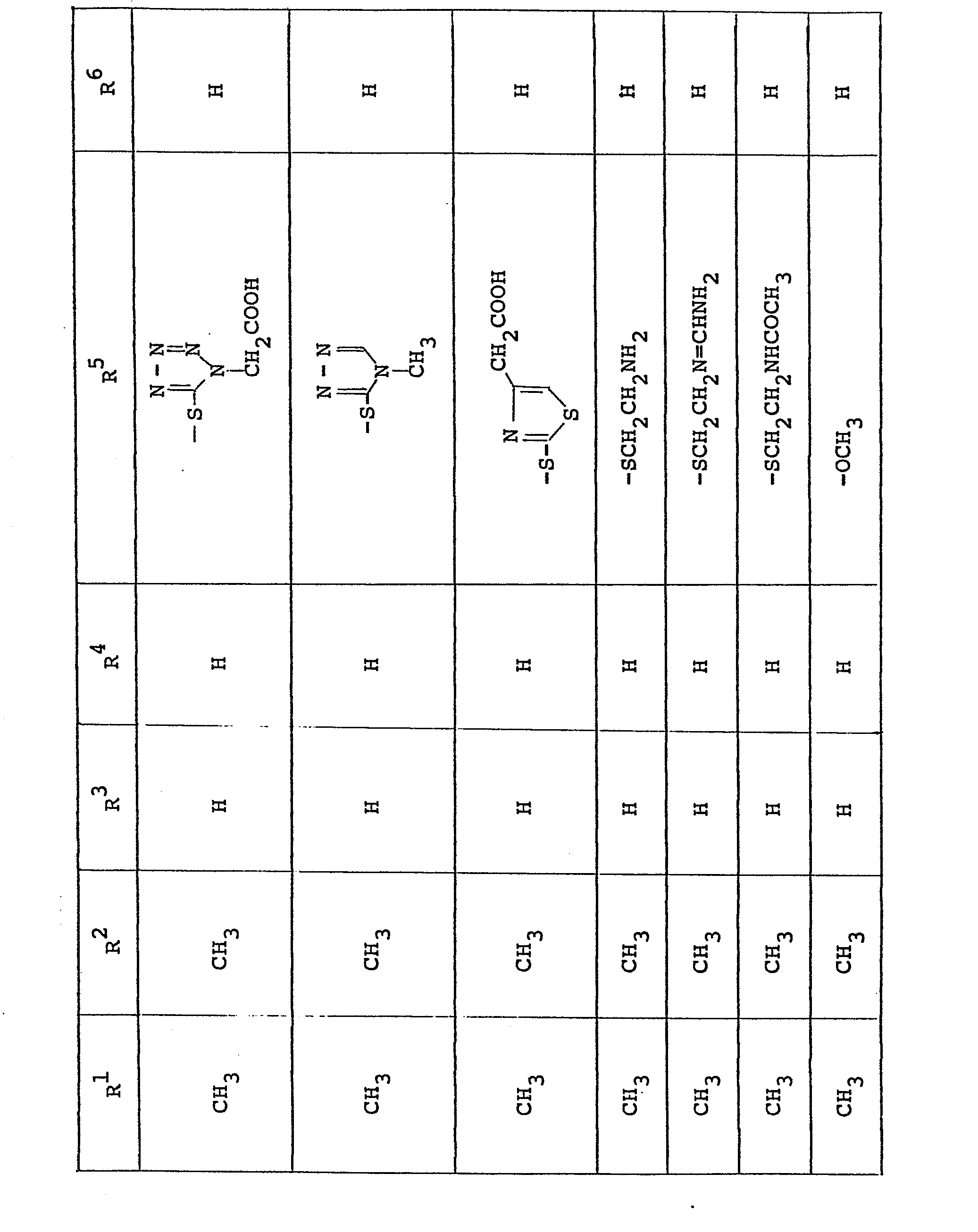

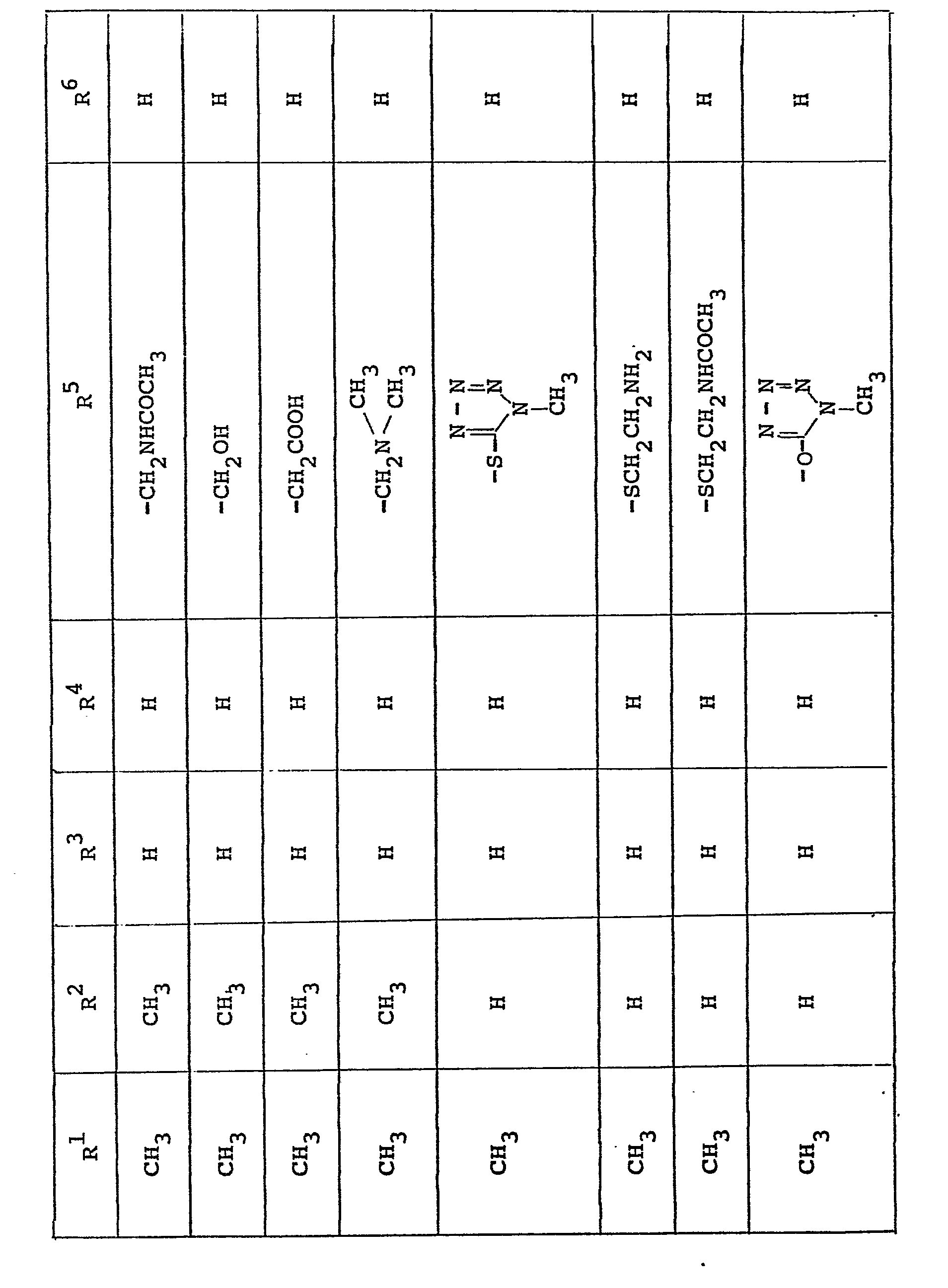

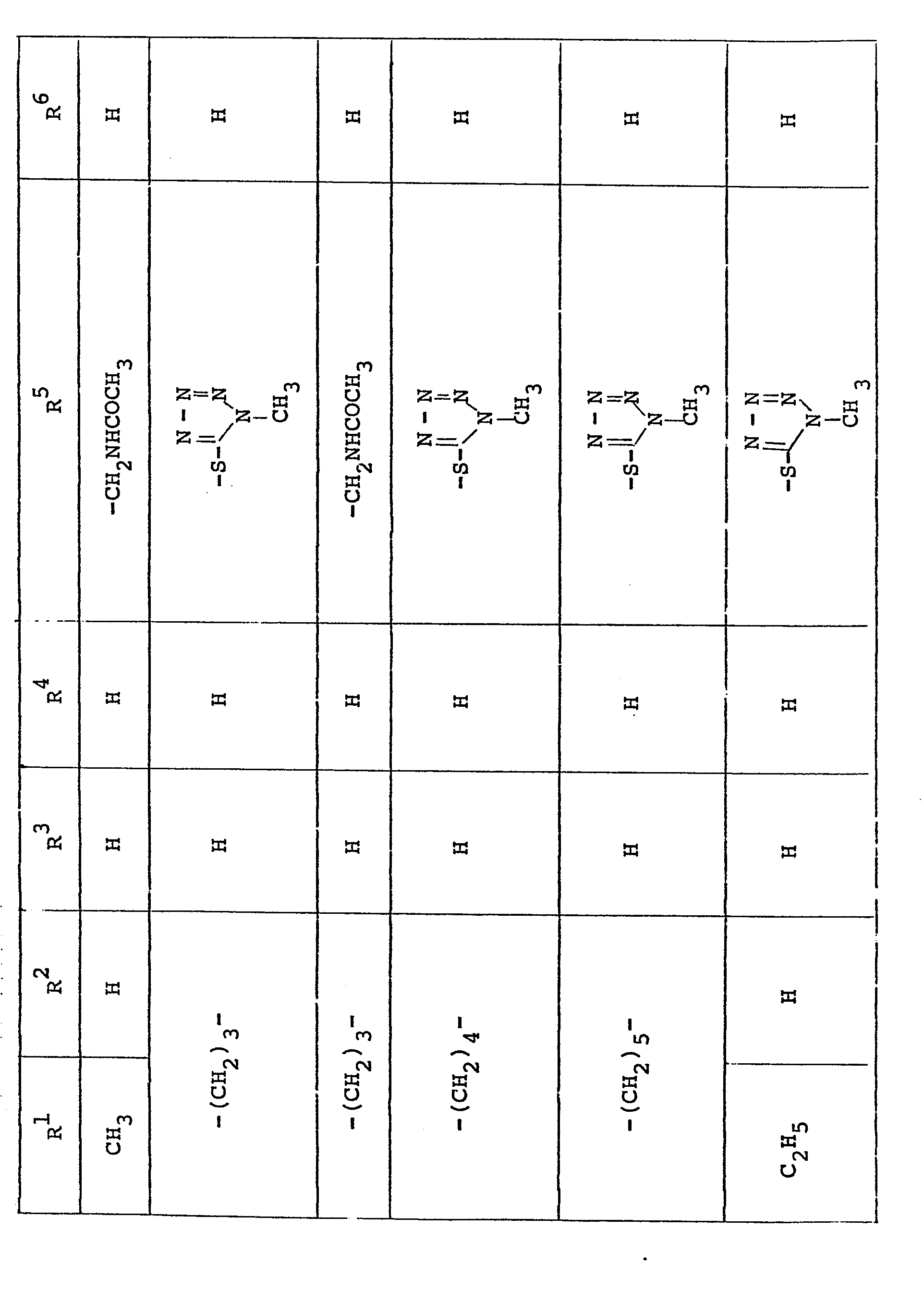

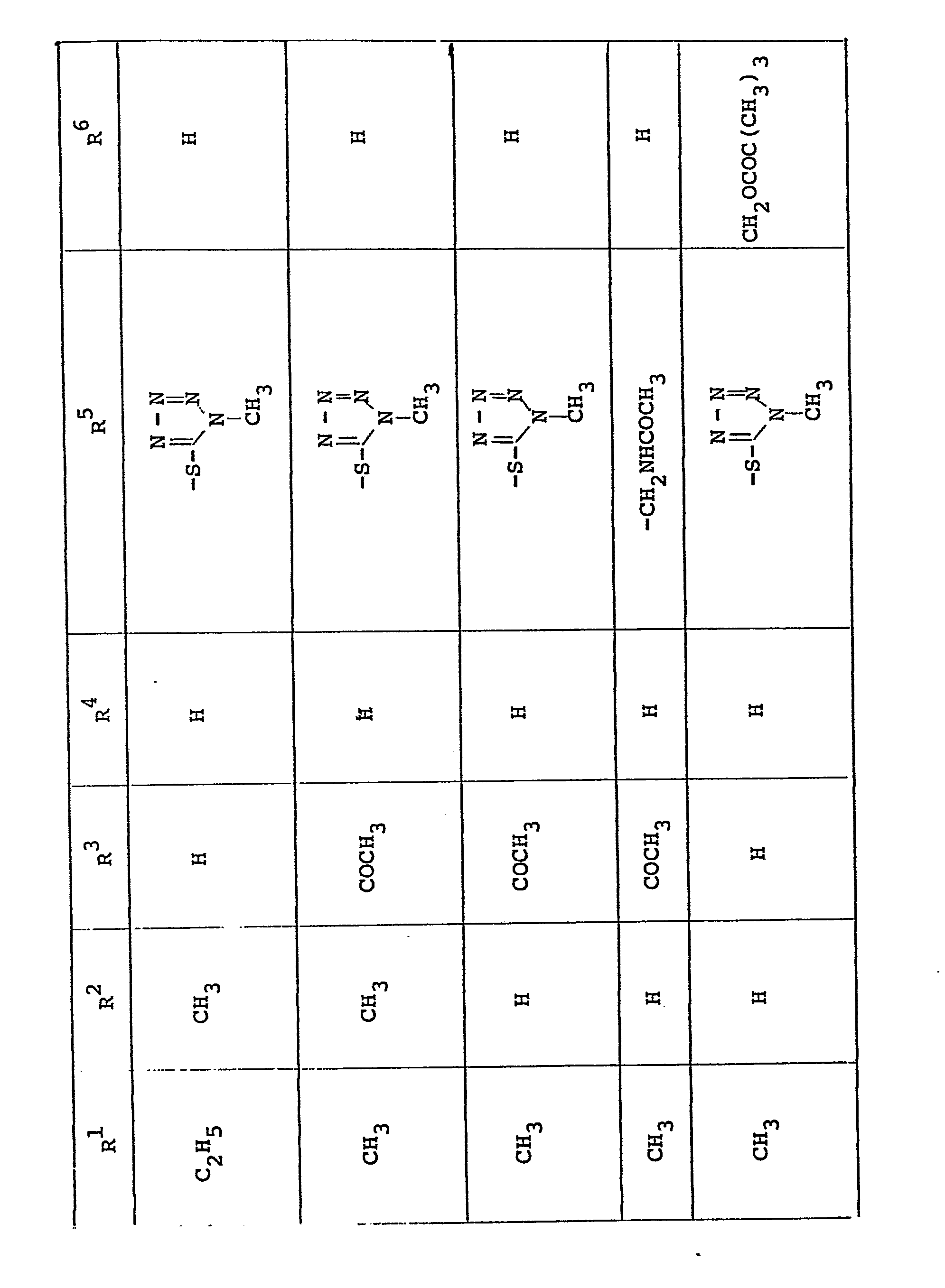

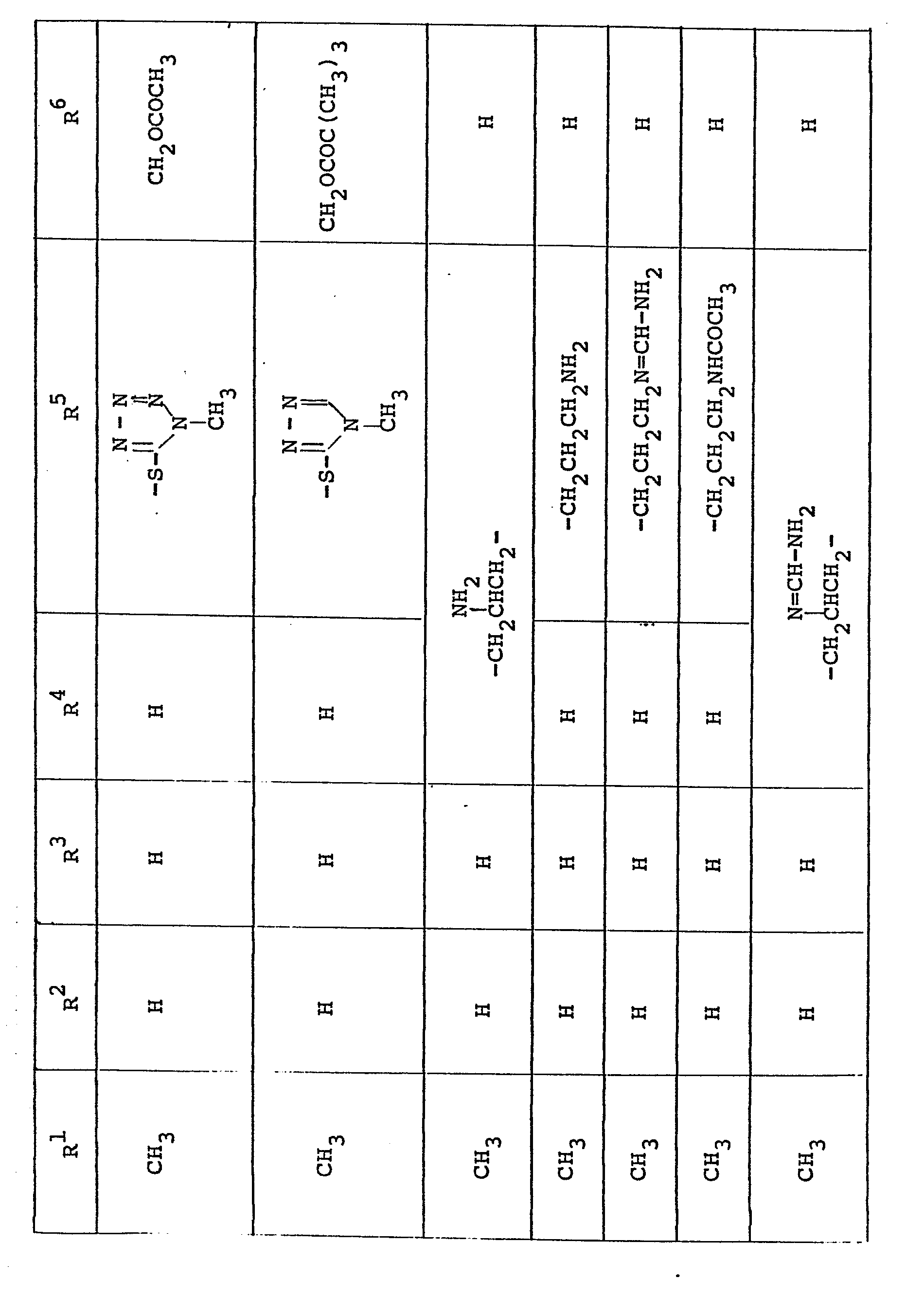

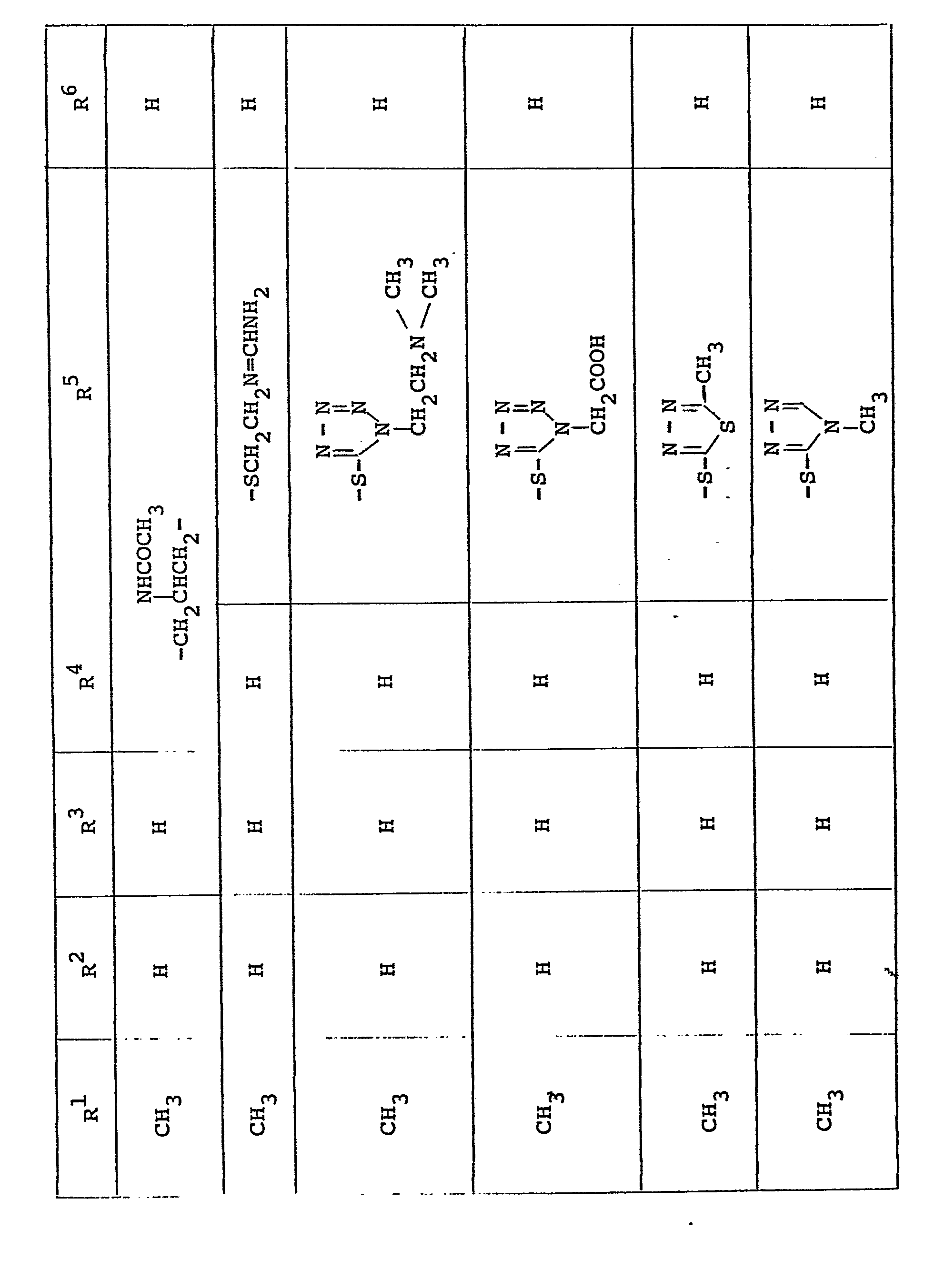

The product compound (I) of this invention can be obtained by cyclizing a compound (II). The group Z of the starting compound (II) is one of the phosphonio or phosphono groups which are well known in connection with the Wittig condensation reaction and particularly is a triaryl (e.g. triphenyl) or tri-lower alkyl (e.g. tributyl)-phosphonio group or a phosphono group diesterified by lower alkyl (e.g. ethyl). [When Z is such a phosphono group, it may contain a still more strongly basic cation, especially a suitable metal ion (e.g. lithium, sodium or potassium ion)]. Referring, further, to the group Z, triphenylphosphonio is preferable in some=cases and a diethylphosphono group accompanying an alkali metal ion (e.g. sodium ion) is preferred in other cases.

The cyclization may be effected spontaneously, that is to say during the production of the starting material compound. Or, it may be effected by heating the compound at a temperature of about 30 to 200°C, preferably about 50 to 150°C. The reaction is preferably conducted in the presence of an inert solvent such as aliphatic, alicyclic or aromatic hydrocarbons, e.g. hexane, cyclohexane, benzene, toluene, xylene or mesitylene, halogenated hydrocarbons e.g. methylene chloride, etc., ethers such as diethyl ether, lower alkylene glycol di-lower alkyl ethers, etc. e.g. dimethoxyethane, diethylene glycol dimethyl ether, etc. cyclic ethers,e.g. dioxane, tetrahydrofuran, etc., carboxylic acid amides, e.g. dimethylformamide, etc. di-lower alkyl sulfoxides,e.g.dimethyl sulfoxide, or lower alkanols,e.g. methanol, ethanol, t-butanol, etc., or mixtures thereof, and if necessary in an inert gaseous atmosphere (e.g. argon, nitrogen gas, etc.). More satisfactory results are often obtained when the reaction is conducted in the presence of a reducing agent such as hydroquinone.

When the amino, hydroxyl or/and carboxy groups of the resulting compound have been protected, these groups are removed if necessary. Amino-protecting groups can be eliminated as follows. t-Butoxycarbonyl, for instance, can be removed with an acid; β,β,β-trichloroethoxycarbonyl with zinc and an acid; monochloroacetyl with thiourea, an N-substituted-dithiocarbamic acid or the like; and p-nitrobenzyloxycarbonyl by catalytic reduction. Elimination of hydroxy-protecting groups can be effected as follows. For example, formyl and trifluoroacetyl can be removed with potassium hydrogen carbonate in aqueous methaol; tetrahydropyranyl with dilute hydrochloric acid, and β,β,β-trichloroethoxycarbonyl with zinc and an acid. Removal of carboxy-protecting groups can be performed in the same manner as that described hereinafter in respect of removal of the protective group R of the carboxyl group at 3-position, and this can be performed simultaneously.

The resulting compound (III) can be separated and purified by conventional procedures such as concentration, pH change, redistribution, solvent extraction, crystallization, recrystallization, chromatography, etc.

The resulting compound (III) can be converted to a carboxylic acid compound by removing the carboxy-protecting group in the conventional manner. While the method of removing the protective group varies with the type of protective group, the group can be eliminated by a procedure generally known in the art. Preferably, the compound (III) in which the substituent group R is a reductively removable protective group such as haloalkyl, aralkyl, benzhydryl or the like is contacted with a reducing agent. The reducing agent used in this reaction is as follows. For example, when the carboxy-protecting group is a haloalkyl group such as 2,2-dibromoethyl or 2,2,2-trichloroethyl, zinc and acetic acid can be suitably employed. When the protective group is an aralkyl group such as benzyl, p-nitrobenzyl or the like or benzhydryl, hydrogen and a catalytic reduction catalyst such as palladium-on-carbon or an alkali metal sulfide such as sodium sulfide or potassium sulfide are suitably employed. o-Nitrobenzyl can be eliminated by irradiation. The reaction is conducted in the:presence of a solvent. The solvent may be any appropriate solvent if it will not interfere with the reaction. Preferred are alcohols such as methanol, ethanol, etc., ethers such as tetrahydrofuran, dioxane, etc., fatty acids such as acetic acid, etc., and mixtures of such organic solvents with water. The reaction temperature.is generally 0°C to near room temperature and the reaction generally goes to completion in 5 minutes to 12 hours, the time required varying with different starting compounds and reducing agents.

After completion of the reaction, the carboxy- deprotected compound can be isolated from the reaction mixture by conventional procedures such as those mentioned hereinbefore. For example, the insolubles are filtered off from the reaction mixture, the organic solvent layer is washed with water, and after drying, the solvent is distilled off.

When Rⁱ and R² in the product (6R)-substituted-(5R)-penem-3-carboxylic acid derivative (I) are different, there exists an isomerism (diastereomers) due to the asymmetric carbon atom C₁,, and while these isomers are shown by the single formula, all of them fall within the scope of this invention. When the substituent groups R and R² are different from each other and R³ is a hydrogen atom [for example, 6-(1-hydroxy-I-ethyl)], the configuration of the hydroxyl group may be changed as desired. Thus, as the compound having a l'-S-hydroxyethyl group as a 6- substituent group, for instance, is treated with an organic acid in the presence of a phosphine derivative such as triphenylphosphine and an azodicarboxylic acid diester such as azodicarboxylic acid diethyl ester, there is obtained the corresponding 1-acyloxyethyl derivative having the R-configuration at position-1' of the 6-side chain. Preferred species of said organic acid are lower fatty acids such as formic acid, acetic acid, propionic acid, etc., and aromatic carboxylic acids and arylalkanoic acids such as benzoic acid, phenylacetic acid, etc. The solvent is preferably a halogenated hydrocarbon such as methylene chloride, chloroform, etc. and ethers such as tetrahydrofuran, dioxane, etc. The reaction temperature preferably ranges from 0°C to 50°C and the reaction time preferably from 10 minutes to 2 hours. This I'-inversion reaction can be performed even before elimination of the carboxy-protecting group of (III).

The product compound (I) may be used in the free form or may be used in the form of a pharmacologically acceptable salt as prepared in the per se known manner. For example,there may be employed such salts as the salts of alkali metals and alkaline earth metals such as lithium,.sodium, potassium, calcium, magnesium, etc., and the ammonium salts such as those of ammonium, dicyclohexylammonium, diisopropylammonium, triethylammonium, etc., although the sodium salt and potassium salt are especially desirable.

When R⁵ contains a basic group such as amino, dimethylamino, formimidoyl or the like, the compound (I) may be converted to and used as the salt of an acid. Examples of such salt include the salts of such mineral acids as hydrochloric acid, sulfuric acid, phosphoric acid, etc., or such organic acids as formic acid, acetic acid, oxalic acid, tartaric acid, citric acid, p-toluenesulfonic acid, etc., although the hydrochloride is a preferred salt.

The compound (I) or salt thereof, which is produced in the above manner, is a valuable antibiotic which is active against a broad spectrum of gram-positive and gram-negative bacteria and is used as a pharmaceutical agent for man and domestic animals. Especially it can be safely used as an antibacterial agent for the treatment of infections caused by gram-positive or gram-negative bacteria such as Staphylococcus aureus, Escherichia coli, Krebsiella ₂neumoniae, etc. Moreover, the antibacterial agent is also incorporated in animal diets as preservatives for preventing putrefaction of feedstuffs. The compound or salt can also be used for discouraging or arresting growth of harmful bacteria on medical or dental equipment and instruments or for inhibiting growth of undesirable bacteria in industrial (especially, water-based) paints and paper mill white waters. For such and other purposes, the compound or salt can be used in aqueous compositions in the concentration of 0.1 to 100 parts per million of each solution.

In various pharmaceutical preparations, the product compound (I) or a salt or ester thereof can be used either singly or in combination with other active components. The dosage forms that can be used include capsules, tablets, powders, solutions, suspensions, elixers, etc. and these preparations can be administered orally, intravenously or intramuscularly.

Tablets for oral administration may contain the common excipients, e.g. binders such'as syrup, gum arabic, gelatin, sorbitol, gum tragacanth, polyvinyl pyrrolidone, etc., fillers such as lactose and other saccharides, corn starch, calcium phosphate, sorbitol, glycine, etc., lubricants such as magnesium stearate, talc, polyethylene glycol, silica, etc., disintegrating agents such as potato starch, and pharmaceutically acceptable wetting agents such as sodium laurylsulfate, etc. These tablets may be coated by the established pharmaceutical procedure. Liquid preparations for oral administration can be made available in such forms as aqueous or oil suspensions, solutions, emulsions, syrups, elixers, etc. or may be dry or lyophilized preparations for extemporaneous dissolution in suitable solvents. Such liquid preparations may contain suspending media such as sorbitol syrup, methylcellulose, glucose sugar syrup, gelatin, hydroxyethylcellulose, carboxymethylcellulose, aluminum stearate gel, etc., hydrogenated edible oils such as almond oil, coconut oil fractions, oily esters, etc., propylene glycol, ethyl alcohol, preservatives such as methyl or propyl p-hydroxybenzoate, etc. or sorbic acid. Suppositories may contain ordinary suppository bases such as cocoa butter and other glycerides.

Injectable compositions may be provided in ampoules or other unit dosage forms containing preservatives. Such compositions may be oil or aqueous suspensions, solutions or emulsions, and may contain such auxiliary agents as suspending agents, stabilizers and/or dispersing agents. Moreover, the active component may be made available in the form of a powder which can be reconstituted with a suitable solvent such as sterile pyrogen-free water before use.

The pharmaceutical preparations according to this invention further include such other suitable forms which permit absorption of the active component from the mucous membranes of the nose and throat or the bronchial tissues, such as powders, liquid spray-mists, or inhalants, lozenge, throat paints, etc. For application to the eye or the ear, the drug can be usedin.the form of a liquid or a semi-solid capsule or a drop. Moreover, the drug according to this invention can be formulated with hydrophobic or hydrophilic bases to provide external preparations such as ointments, creams, lotions, paints, powders. etc.

Aside from or in addition to carriers and vehicles, there may be incorporated stabilizers, binders, antioxidants, preservatives, lubricants, suspending agents, thickeners, flavorants, etc. Moreover, it is possible to incorporate other active components in compositions to provide a broader antibacterial spectrum.

For domestic animals, the drug according to this invention can be formulated as intramammary preparations using slow-release or quick-release media.

The dosage depends on the condition of the patient to be treated or the body weight of the host animal, the method and frequency of administration, and the route of administration, e.g. parenteral and other routes preferred for general infections and the oral route for alimentary tract infections. Generally, the daily oral dose is about 15 to 600 mg of the active component per kg body weight of patient in an administration schedule of once daily or higher frequency. A preferred daily dose for an adult man is about 80 to 120 mg as active component per kg body weight.

The compositions of this invention can be administered in various unit dosage forms such as solid or liquid forms which can be orally taken. The composition of such a solid or liquid unit dosage form may include 0.5 to 99%, preferably about 10-60% of the active component. The composition generally contains about 15 to 1500 mg of the active component. However, the preferred amount is about 250 to 1000 mg.

Since the compound, salt or ester according to this invention has β-lactamase-inhibiting activity, it can be used in combination with a-lactam antibiotics. Such α-lactam antibiotics include, among others, penicillin antibiotics such as benzylpenicillin, phenoxymethylpenicillin, carbenicillin, ampicillin, amoxicillin, sulbenicillin, etc. and cephalosporin antibiotics such as cephaloridine, cephalo- thin, cefazolin, cefalexin, cefoxitin, cephacetrile, cefa- mandole, cefmetoxime, cefsulodin, cefotiam, cefotaxime, cefapirin, ceftizoxime, cefradine, cefaloglycin, etc.

The starting compound of the formula (II) can be produced for example in accordance with the following reaction schema. 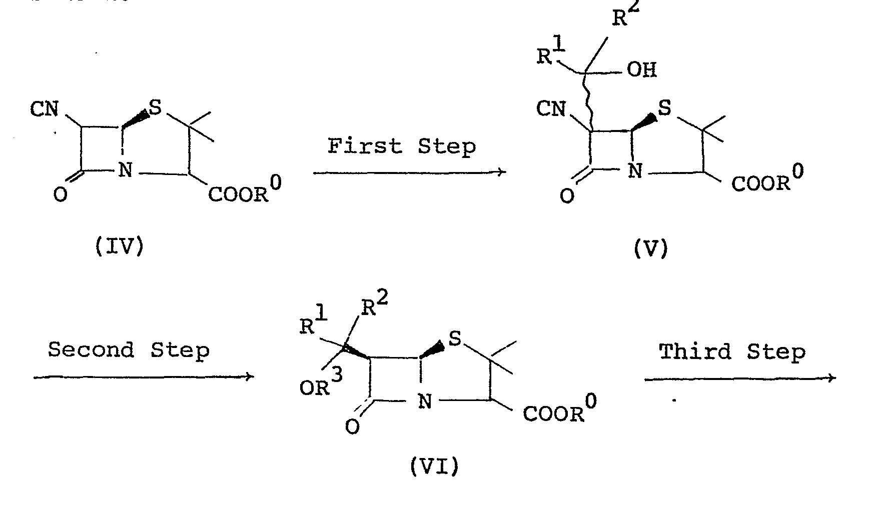 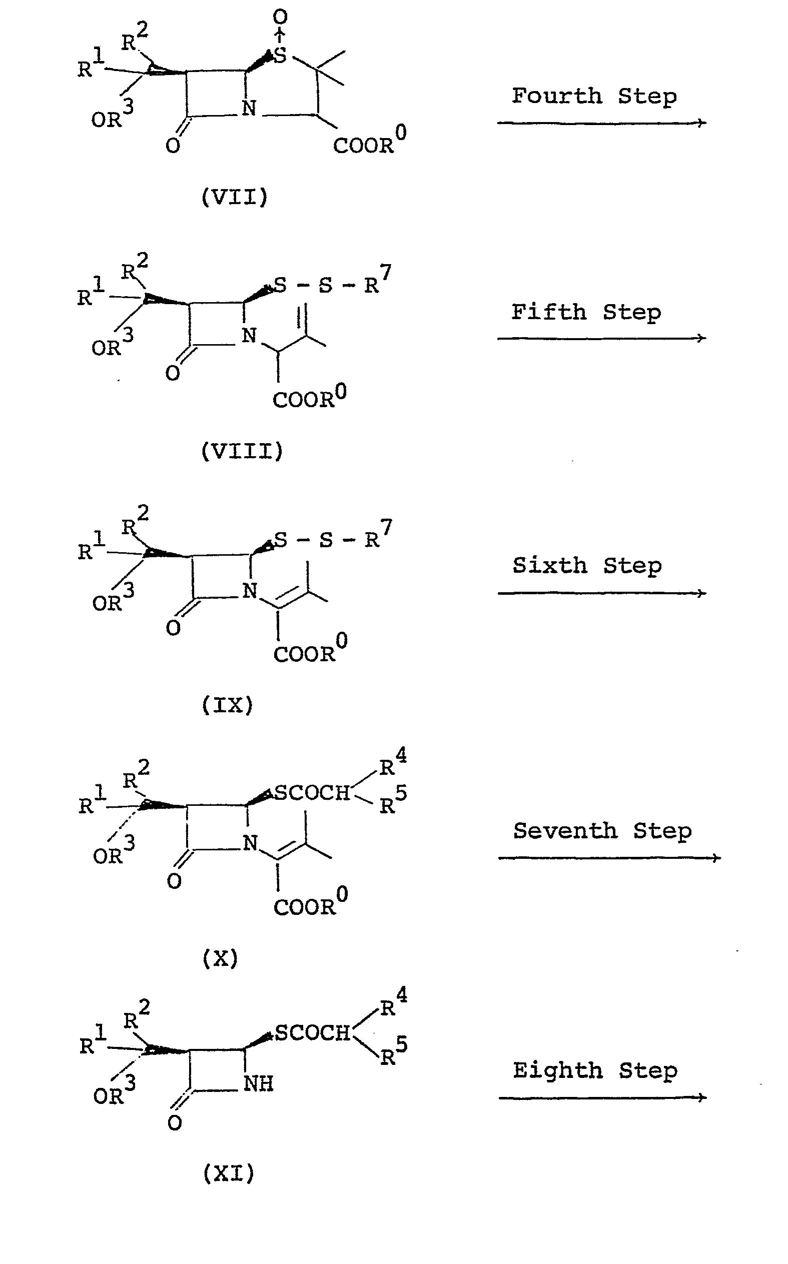 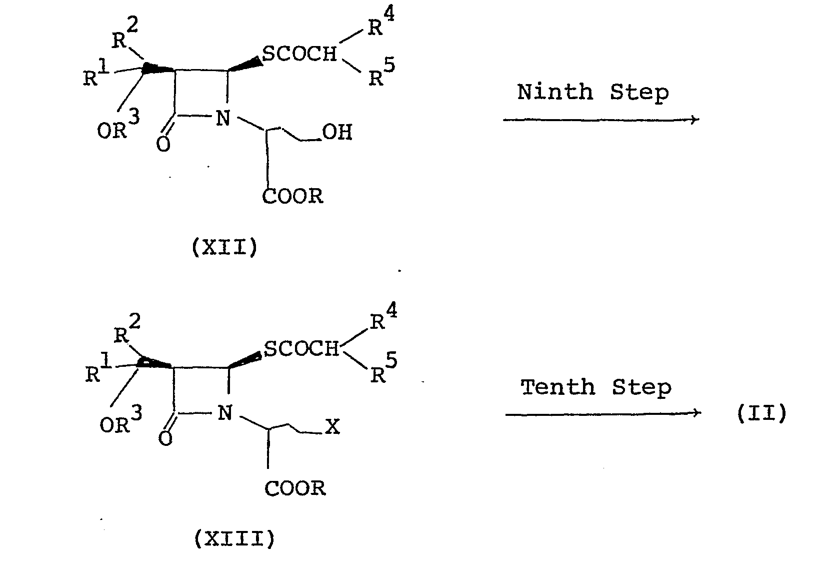 wherein _{R}^{l}, _{R}², R³, R4 and R⁵ have the same meanings as defined hereinbefore; R is a carboxy-protecting group; R is a hydrocarbon group or a heterocyclic group; X is a halogen atom or an organic sulfonyloxy group.

### First Step

This is a process for the production of a compound of formula (V), wherein a compound of formula (IV) is reacted with a carbonyl compound of formula (XIV):
R¹-CO-R² (XIV) wherein R^{l} and R² have the same meanings as defined hereinbefore, in the presence of a base. The said 6-isonitrilepenicillin of formula (IV) which is used as a starting compound in this process can be produced by the process described in International Patent Application PCT/JP80/00281.

The reaction is accomplished by contacting compound (IV) with at least a mole equivalent of compound (XIV) in the presence of a base. The solvent used for this reaction may be either the compound (XIV) or a different solvent that will not interfere with the reaction. Such a solvent is preferably one that will dissolve both compounds (IV) and (XIV). Examples of the solvent include alcohols such as methanol, ethanol, n-propanol, etc., dialkyl alkanoic acid amides such as dimethylformamide, dimethylacetamide, etc., cyclic ethers such as tetrahydrofuran, dioxane, etc., ethers such as ethyl ether, diglyme, dimethoxyethane, etc., polar aprotic solvents such as dimethyl sulfoxide, hexamethylphosphoric acid triamide, etc. and mixtures of water with such organic solvents: The base mentioned above may be a virtually optional type only if it is capable of even a temporal release of an anion at 6-position of compound (IV). However, it is important to select a suitable combination thereof with the solvent. When a protic solvent is employed, there is employed an inorganic base such as potassium carbonate, sodium carbonate, sodium hydroxide, potassium hydroxide, etc. When an aprotic solvent is employed, there may be suitably employed n-butyllithium, methyllithium, diethyl sodium amide, diisopropyl sodium amide, isopropyl- cyclohexyl sodium amide, ditrimethylsilyl sodium amide, etc. Triethylamine, diethylisopropylamine, diethylamine, diisopropylamine, dicyclohexylamine, l,5-diazabicyclo[4,3,0]-non-5-ene, 1,8-diazabicyclo[5,4,O]-7-undecene, etc. may suitably be employed in both an aprotic solvent and a protic solvent. While the reaction temperature and time are not especially critical, it depends mainly on the species of starting compounds (IV) and (XIV), solvent and base employed. Generally, the reaction is conducted at -78°C to +50°C for 1 to 14 hours.

After completion of the reaction, the product compound (V) of this reaction is recovered from the reaction mixture in the conventional manner. For example,a water-immiscible organic solvent such as ethyl acetate and water are added to the reaction mixture, the organic layer is separated then washed with water and dried over a desiccant and the solvent is distilled off to recover the desired compound.

The product compound thus produced can be further purified, if necessary, by the conventional procedure such as recrystallization,preparative thin-layer chromatography, column chromatography, etc.

### Second Step

In this step, the compound (V) is subjected to reductive removal of isocyano group to selectively give a cis- oriented 6-hydroxyalkylpenicillin derivative (VI) and, if necessary, the latter derivative is reacted with an acylating agent or a hydroxy-protective group-forming reagent. The above two reactions may be performed in.a reverse order. That is to say, the acylation or protection of the hydroxy group may be first performed and the removal of isocyano group then performed to obtain the desired compound (VI). This reductive removal of isocyano group is accomplished by contacting the compound (V), or the compound (V) whose hydroxy group has been acylated or protected, with a reducing agent such as tri-n-butyltin hydride in the presence of a radical reaction initiator such as benzoyl peroxide, azobisisobutyronitrile or the like. Since this is a radical reaction, it is preferably carried out in an oxygen- and air-free atmosphere, that is to say in a nitrogen or argon gas atmosphere, for instance. The type of solvent used for this reaction is not particularly critical insofar as it does not interefere with the reaction but generally benzene, toluene, xylene, acetone and the like are employed. The reaction temperature is not especially critical, either, but generally ranges from about 40°C to about 100°C. While the reaction time varies with different species of starting compounds, reaction temperature, species of solvent, etc., it is generally in the range of 2 minutes to 24 hours.

After completion of the reaction, the product compound can be isolated from the reaction mixture in the conventional manner. For example, the solvent is first removed from the mixture and if necessary, the excess reagents and side reaction byproducts are removed by recrystallization and column chromatography.

The acylation or protection of the hydroxy group can be accomplished by the conventional method used in the field of organic chemistry. Since the acylation reaction results in the introduction of the acyl group as R of compound (I) and, hence, influences the antibacterial and 8-lactamase-inhibiting activities of (I), care should be exercised in the selection of the type of acyl group. Accordingly, for example, active derivatives of aliphatic carboxylic acids such as acetic acid, propionic acid, n-butyric acid, isobutyric acid, etc., arylcarboxylic acids such as benzoic acid, etc., or aralkylcarboxylic acids such as phenylacetic acid, etc. e.g. the corresponding acid halides, acid anhydrides, mixed acid anhydrides, active esters, etc. are employed.

The protection reaction of the hydroxyl group is preferably carried out with a reagent that is commonly used in the field of penicillins and cephalosporins, for the deprotection reaction is conducted for the purpose of regenerating the hydroxy group under mild conditions. Examples of such reagents are silylating agents such as tert-butyldimethylsilyl chloride, trimethylsilyl chloride, etc., chloroformic acid benzyl esters such as p-nitrobenzyl chloroformate, o-nitrobenzyl chloroformate, etc., methoxyethyl chloride, tetrahydropyran, etc. The acylation or protection of the hydroxy group with the above-mentioned reagents is sometimes accelerated in the presence of a base.

The acylation or protection reaction of the hydroxy group may be carried out before or after any optional one of the third to tenth steps which are described hereinafter but since the same principle of the reaction as set forth above is applicable, there will not be reiterated in the following description.

### Third Step

This is a process in which compound (VI) is oxidized to compound (VII).

This reaction is accomplished by contacting compound (VI) with an oxidizing agent in a solvent. The preferred oxidizing agent includes, for exmaple, peracids such as m-chloroperbenzoic acid, perbenzoic acid, peracetic acid, etc., hypochlorous acid esters such as t-butyl hypochlorite, i-amyl hypochlorite, etc., salts of hypochlorous acid such as sodium hypochlorite, potassium hypochlorite, etc., permanganates such as potassium permanganate, sodium permanganate, etc., periodates such as sodium metaperiodate, etc., ozone, and so on. Since the use of a large excess of the oxidizing agent in this reaction may result in the production of the sulfo compound, it is preferable to use an equivalent or a slight excess thereof. The type of solvent is virtually optional only if it will not undergo oxidation. When an organic oxidizing agent is employed, such solvents as chloroform, dichloromethane, dichloroethane, etc., are preferred in many cases because they dissolve compound (VI) and oxidizing agent well. It is also possible to employ water in combination with other solvents. To avoid by-production of the sulfo compound, generally the reaction temperature is preferably low. Usually, the reaction is conducted at -20°C to +30°C. The reaction time depends largely on the species of starting compound, oxidizing agent and solvent employed, the reaction temperature, etc., and generally ranges from about 10 minutes to about 24 hours.

After completion of the reaction, the product compound (VII) of this reaction step is isolated from the reaction mixture in the conventional manner. When, for example, a peracid is used as the oxidizing agent, the reaction mixture is washed with an alkaline aqueous solution to remove the excess reagent and after drying, the solvent is distilled off. The compound (VII) may exist as isomers with respect to the sulfoxide. Thus, (VII) exists as a mixture of four isomers when R¹ and R² are different from each other and as a mixture of two isomers when R¹ and R² are identical. If necessary, these isomers can be independently isolated by such procedures as recrystallization, column chromatography. Fourth Step

In this step, compound (VII) is reacted with a thiol compound of the formula 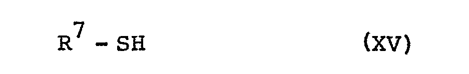 wherein R⁷ is an alkyl, aryl, aralkyl or heterocyclic group, to give compound (VIII).

This reaction is generally conducted in a solvent. The solvent includes, among others, benzene, toluene, xylene, tert-butanol, isopropyl alcohol, methyl isobutyl ketone, methyl ethyl ketone, dioxane, dimethylformamide, etc., mixtures of such solvents, and other solvents which will not interfere with the reaction.

While the reaction temperature is not critical, it is preferably within the range of 50 to 150°C. The reaction is generally carried outin.the neighborhood of the boiling point of the solvent used and goes to completion in 0.5 to 20 hours. After completion of the reaction, the solvent is distilled off and the product compound is isolated generally by silica gel column chromatography.

The product compound may be used in the next reaction without prior purification.

Examples of R in compound (XV) are as follows. (I) Alkyl groups such as methyl, ethyl, propyl, isopropyl, butyl, isobutyl, etc., (II) unsubstituted or substituted aryl groups such as phenyl, xylyl, tolyl, naphthyl, chlorophenyl, nitrophenyl, bromophenyl, etc., (III) unsubstituted or substituted aralkyl groups such as benzyl, phenethyl, phenylpropyl, xylyl- methyl, p-chlorobenzyl, o-nitrobenzyl, p-nitrobenzyl, p-methoxybenzyl, 2,4-dimethoxybenzyl, 2-chloro-4-methoxybenzyl, etc.; (IV) aliphatic or aromatic heterocyclic monocyclic groups or heterocyclic polycyclic groups containing one or more same or different hetero-atoms, i.e. oxygen, nitrogen and sulfur atoms, such as pyrrolidinyl, piperazinyl, homo- piperidinyl, furyl, thienyl, pyrrolyl, pyridyl, imidazolidinyl, benzothiazolyl, benzoxazolyl, thiazolyl, thiatriazolyl, oxazolyl, oxadiazolyl, oxatriazolyl, triazolyl, tetrazolyl, etc., and such heterocyclic groups having one or more same or different substituents such as alkyl groups, e.g. methyl, ethyl, etc., alkoxy groups, e.g.methoxy, ethoxy, etc., halogen atoms, e.g. fluoro, chloro, bromo, etc., nitro, aryl groups, e.g. phenyl, tolyl, xylyl, etc., substituted aryl groups, e.g. chlorophenyl, nitrophenyl, etc., and aralkyl groups, e.g. benzyl, phenethyl, etc.

### Fifth Step

This is a step in which compound (VIII) is treated with a base to isomerize its double bond and to thereby give compound (IX) .

This reaction is accomplished by contacting compound (VIII) with a base in the presence of a solvent. The base used for this purpose may be virtually of any type only if it is able to withdraw the a-hydrogen from the alkoxycarbonyl group of (VIII), although triethylamine is generally employed. There is no particular limitation on the type of solvent only if it does not interfere with the contemplated reaction but, generally, chloroform, dichloromethane, dichloroethane, etc. are employed. There are no particular limits to the reaction temperature and the reaction is generally conducted at 0°C to near room temperature. However, to avoid coloration of the reaction mixture, the reaction is preferably conducted in the neighborhood of 0°C. Thus, generally, the reaction is conducted in dichloromethane using an equivalent of triethylamine at 0°C for about 1 hour. After completion of the reaction, the triethylamine is not distilled off but neutralized with an acid such as hydrochloric acid, formic acid, acetic acid, etc. This procedure is preferred'because the reaction mixture is protected against coloration. The product compound (IX) can be further purified, if necessary, by the conventional procedures such as recrystallization, preparative thin-layer chromatography, column chromatography, etc.

### Sixth Step

This is a step where compound (IX) is reacted with an active derivative of a carboxylic acid of the formula: 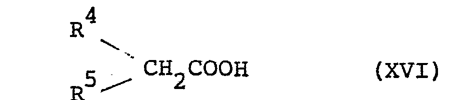 wherein _{R}⁴ and R⁵ have the meanings defined hereinbefore, in the presence of a trivalent phosphorus compound and water to give compound (X).

This reaction is accomplished by contacting compound (IX) with an active derivative of carboxylic acid (XVI) in the presence of water and a trivalent phosphorus compound. The solvent used for this reaction may be any solvent that is compatible with the water, trivalent phosphorus compound and active derivative of carboxylic acid (XVI). Generally, however, chloroform, dichloromethane, dichloroethane, ethyl ether, tetrahydrofuran, dioxane, ethyl acetate, benzene, toluene, hexane and other nonpolar aprotic solvents are preferably employed. Preferred examples of said trivalent phosphorus compounds are phosphorous acid esters such as trimethyl phosphite, triethyl phosphite, tripropyl phosphite, triphenyl phosphite, etc., and phosphines such as triphenylphosphine, etc. The active derivative of carboxylic acid (XVI) may for example be the acid chloride, acid bromide, acid anhydride, mixed acid anhydride, active esters or the like. There are no particular limits to the reaction temperature but the reaction is preferably conducted under cooling or at room temperature. For the purpose of accelerating the reaction as well as when an acid is byproduced depending on individual reactions, a base such as sodium hydrogen carbonate, sodium carbonate, potassium hydrogen carbonate, potassium carbonate, sodium hydroxide, potassium hydroxide, calcium hydroxide, pyridine, picoline, collidine, lutidine, aniline, dimethylaniline, diethylaniline, etc. is often allowed to be present in the reaction system so as to neutralize the acid. In view of decomposition with the water present in the system, it is desirable to employ the active carboxylic acid derivative in excess of a molecular equivalent.

After completion of the reaction, the product compound (X) of this reaction step can be separated from the reaction mixture in the conventional manner and, if necessary, further purified by such procedures as recrystallization, preparative thin-layer chromatography, column chromatography, etc.

There are cases in which compound (X) can be synthesized from compound (VII) by a different route of synthesis as shown below by reaction formulas but it must be noted that the compound of formula (XVIII) is sometimes produced as a dominant reaction product.

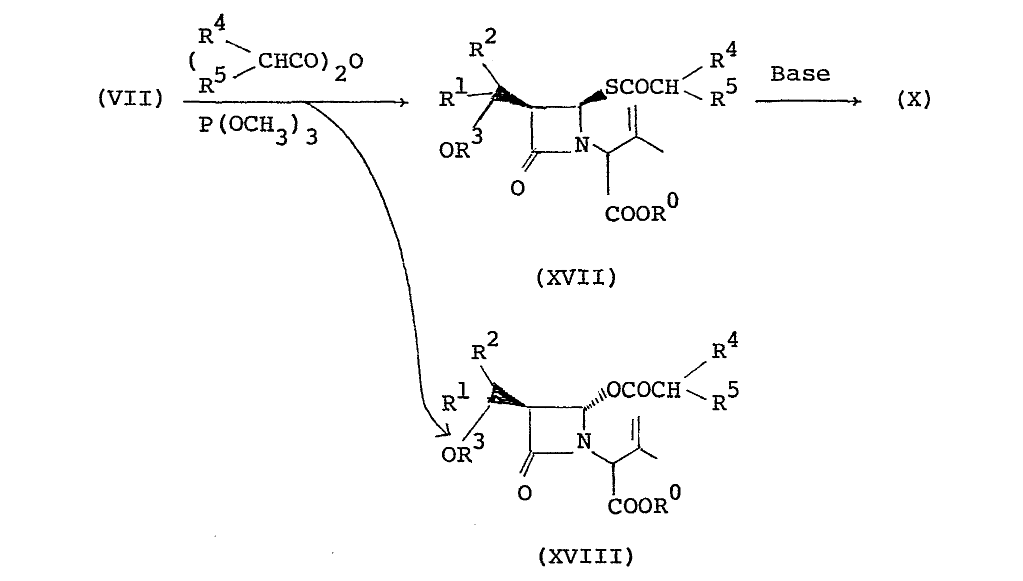

### Seventh Step

In this step, compound (X) is subjected to ozonolysis and the resulting a-keto-carboxylic acid derivative is solvolyzed to compound (XI).

The first reaction of this step is accomplished by contacting compound (X) with ozone in a solvent. The solvent may be any solvent only if it dissolves (X), does not react with ozone and does not solidify at low temperature. Generally, however, methanol, ethanol, ethyl acetate, dichloromethane, chloroform, etc. are suitably employed.

The reaction is preferably conducted at a low temperature, i.e. preferably at -78°C to -30°C. The reaction time depends largely on the species of starting compound and solvent used, the reaction temperature and the capacity of the ozone generator used but the reaction is complete in one minute to one hour under a constant excess supply of ozone.

After completion of the reactoin, the product a-keto-carboxylic acid derivative of the first phase of this process can be recovered from the reaction mixture in the per se conventional manner. For example, an inert gas such as nitroben, argon gas, etc. is introduced into the reaction mixture to drive out the excess ozone and, then, the mixture is contacted with a sulfide such as dimethyl sulfide, diethyl sulfide, etc. or a reducing agent such as sodium hydrogen sulfite, sodium thiosulfate, etc., followed by the conventional separation and purification procedure to recover the a-keto-carboxylic acid derivative. Generally, this a-keto-carboxylic acid derivative is subjected to the second phase of this reaction process without further purification.

The reaction in the second phase of this process is accomplished by subjecting the above a-keto-carboxylic acid derivative to solvolysis. The solvents which are preferably employed for this solvolysis reaction include polar protic solvents such as methanol, ethanol, propanol, butanol, etc. However, virtually any solvent is sufficient only if it does not interfere with the reaction. The addition of a catalytic amount of such an agent as sodium hydroxide, potassium hydroxide, sodium methoxide, sodium ethoxide, etc. for accelerating the reaction leads to satisfactory results in many instances. Silica gel may also be used for the same purpose. While there are no particular limits to the reaction temperature, the reaction is preferably conducted in the neighborhood of room temperature. The reaction time depends largely on the species of starting compound and solvent, the presence, amount and type of catalyst, and the reaction temperature, the reaction generally goes to completion in one to 24 hours provided that methanol is used as the solvent, sodium methoxide present and the reaction conducted at room temperature.

After completion of the reaction,the product compound (XI) of this reaction step is separated from the reaction mixture in the per se conventional manner. For example, the solvent is distilled off from the reaction mixture and the residue redissolved in a water-immiscible solvent, followed by aqueous washing, drying and distillation for removal of the solvent. The product compound thus obtained contains the byproduct oxalic acid ester and, if necessary, can be further purified by the per se conventional procedure such as recrystallization, preparative thin-layer chromatography, column chromatography, etc.

### Eighth Step

In this step, a compound of the general formula (XII) is produced. Here, the compound (XI) is subjected to addition reaction with a glyoxylic acid ester derivative of the general formula: 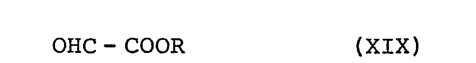 wherein R has the meaning defined hereinbefore.

This reaction is accomplished by contacting compound (XI) with compound (XIX) in the presence of a solvent. While the solvent may be virtually any solvent only if it does not interfere with the reaction, ethers such as tetrahydrofuran, dioxane, etc., aromatic hydrocarbons such as benzene, toluene, etc., dialkylalkanoic acid amides such as dimethylformamide, dimethylacetamide, etc., and mixtures of such organic solvents are preferred. This addition reaction is sometimes accelerated in the presence of a base. Examples of the base used for the purpose include organic bases such as triethylamine, diisopropylethylamine, pyridine, etc., aluminum sodium silicate molecular sieve, etc. The reaction temperature is not particularly critical and may range from room temperature to about 120°C. Preferably, the reaction is conducted in the neighborhood of room temperature when the base is used, and under heating at a temperature near the reflux point of the solvent when the base is not employed. If necessary, the reaction may be conducted in an inert gas atmosphere such as nitrogen, etc. The reaction time depends largely on the species of starting compounds, the reaction temperature, etc. but generally ranges from about 1 hour to about 96 hours.

After completion of the reactoin, the product compound (XII) of this reaciton step is separated from the reaction mixture in the per se conventional manner. For example, the insolubles are first filtered off if necessary. Then, the residue is washed with water and dried, followed by distillation for removal of the solvent and excess reagents.

Where R of compound (X) and R of compound (XII) represent the same group, (XII) can be synthesized from (X) by a different route of synthesis as depicted below.

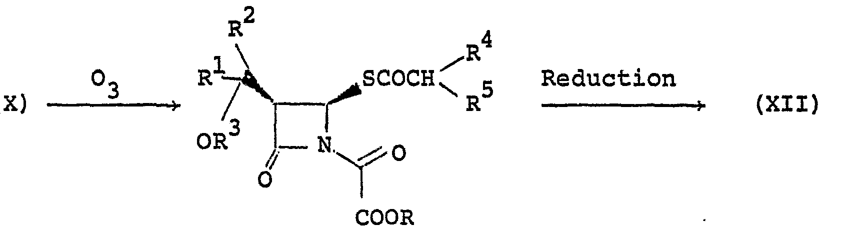

### Ninth Step

In this step, compound (XII) is halogenated to give compound (XIII).

This reaction is accomplished by contacting compound (XII) with a halogenating agent in the presence of a solvent. While the type of solvent for this purpose is not particularly critical, thionyl halides such as thionyl chloride, thionyl bromide, etc., phosphorus oxyhalides such as phosphorus oxychloride, etc., phosphorus halides such as phosphorus pentachloride, phosphorus pentabromide, etc., and oxalyl halides such as oxalyl chloride may be employed with advantage. This reaction is preferably conducted in the presence of a base. Preferred species of said base are organic bases such as triethylamine, diisopropylethylamine, pyridine, lutidine, etc. The type of solvent used for this reaction is not particularly critical but ethers such as tetrahydrofuran, dioxane, etc. are preferred. The reaction temperature is not very critical, either, but is preferably low so that side reactions will be inhibited. Thus, the reaction is preferably conducted in the range of -40°C to room temperature. If necessary, the reaction may be conducted in an inert gas, e.g. N₂, atmosphere. The reaction time depends largely on the species of starting compounds, the reaction temperature, etc. and ranges generally from about 10 to 30 minutes.

After completion of the reaction, the product compound (XIII) of this reaction step is separated from the reaction mixture in the per se conventional manner. For example, the solvent and excess reagents are distilled off from the reaction mixture to give (XIII). Generally, this compound is used in the next reaction without further purification.

Referring to this product compound (XIII), the halogen atom X can be replaced with a different halogen atom by a conventinoal method. For example, the corresponding chloro compound is treated with an inorganic bromide or iodide salt such as lithium bromide, potassium iodide, etc., in an organic solvent such as dimethylformamide, dimethylacetamide or dimethyl sulfoxide to give the bromo or iodo compound.

### Tenth Step

This is a process for the production of (II), wherein compound (XIII) is converted to a phosphorane compound.

This reaction is accomplished by contacting compound (XII) with a phosphine compound or a phosphorous acid ester compound and a base in a solvent. The phosphine compound may for example a tri-lower alkylphosphine such as tri-n-butylphosphine or a triarylphosphine such as triphenylphosphine. The phosphorous acid ester compound is preferably selected from among tri-lower alkyl phosphites such as triethyl phosphite, etc. and di-lower alkyl phosphite alkali metal salts such as sodium dimethyl phosphite. The base is preferably an organic base such as triethylamine, diisopropylethylamine, pyridine, 2,6- lutidine, etc. when a phosphine compound is employed, and an alkali metal hydride such as a sodium hydride or a lower alkyllithium compound such as n-butyllithium when a phosphorous acid ester compound is employed. The solvent used for this reaction is virtually of any type only if it will not interfere with the reaction but is preferably selected from among aliphatic hydrocarbons such as hexane, cyclohexane, etc., ethers such as tetrahydrofuran, dioxane etc., aromatic hydrocarbons such as benzene, toluene, etc., and dialkylalkanoic acid amides such as dimethylformamide, dimethylacetamide, etc. while the reaction temperature is not particularly critical, the reaction is preferably conducted generally in the range of room temperature to 100°C. If necessary, this reaction may be conducted in an inert gas atmosphere such as nitrogen, etc. The reaction time depends mainly on the species of starting compounds, the reaction time, etc. but ranges form about 1 to 20 hours.

After completion of the reaction, the product compound (II) of this reaction step is separated from the reaction mixture in the per se conventional manner. For example, water and a water-immiscible organic solvent such as ethyl acetate are added to the reaction mixture, the organic layer is separated, washed with water and dried over a desiccant and the solvent is distilled off to give (II).

The product compound (II), if necessary, can be purified by the per se conventional procedure such as recrystallization, preparative thin-layer chromatography, column chromatography, etc.

The follwing reference and working examples are given to further illustrate this invention but should by no means be construed as limiting its scope. In the examples, Me means methyl; Bu, butyl; But, tert-butyl; AcOEt, ethyl acetate; AcOH, acetic acid; s, a singlet; d, a doublet; m, a multiplet; ABq, an A-B pattern quartet; br, broad; dd, a double doublet; q, a quartet; mp, melting point; dec., decomposition; and TLC, thin-layer chromatography on a Merck thin-layer chromato- plate Art. 5715.

### Reference Example 1

### Pivaloyloxymethyl 2-[(3R,4R)-4-(benzothiazol-2-yldithio)-3-(1-hydroxyisopropyl)-2-oxoazetidin-1-yl]-3-methylcrotonate

1) To 3.583 g of pivaloyloxymethyl 6β-(1-hydroxyisopropyl)penicillanate 1-oxide are added 71.7 ml of toluene and 1.54 g of 2-mercaptobenzothiazole and the mixture is stirred under heating for 3 hours, with the byproduct water being removed with a Dean-Stark trap. The toluene is then distilled off under reduced pressure to give pivaloyloxymethyl 2-[(3R,4R)-4-(benzothiazol-2-yldithio)-3-(l-hydroxyisopropyl)-2-oxoazetidin-l-yl]-3-methylbutyrate in quantitative yield.
2) The above ester thus obtained is dissolved in 71.7 ml of methylene chloride, and following addition of 1.29 ml of triethylamine, the mixture is stirred at room temperature for 50 minutes. The solvent is then distilled off under reduced pressure. The above-identified compound is obtained as a frothy or syrupy substance in quantitative yield.

IR(KBr): 1752, 1430, 1130, 1078, 1004, 986 cm⁻¹ OH NMR(CDCl₃) δ: 1.16s(Bu^{t}), 1.60s(Me₂C-), 2.03s & 2.09s 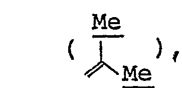 2.95br(OH), 3.64d(J=5Hz,C₃-H), 5.41d(J=5Hz,C₄-H), 5.67 & 5.83ABq(J=6Hz, -OCH₂O), 7.2-7.6m(2H) & 7.7-8.0m(2H, aromatic protons). Rf [AcOEt-hexane (1:1)]: 0.61

### Reference Example 2

### Pivaloyloxymethyl 2-[(3R,4R)-4-acetylthio-3-(acetylthio-3-(1-hydroxyisopropyl)-2-oxoazetidin-1-yl]-3-methylcrotonate

1) In 10 ml of toluene is suspended 389 mg of pivaloyloxymethyl 6β-(1-hydroxyisopropyl)penicillanate 1- oxide. To the suspension are added 0.471 ml of acetic anhydride and 0.118 ml of trimethyl phosphite in that order and the mixture is stirred under heating at 110°C for 3 hours and 40 minutes. The solvent is then distilled off under reduced pressure, and the residual pivaloyloxymethyl 2-[(3R,4R)-4-acetylthio-3-(1-hydroxyisopropyl)-2- oxoazetidin-1-yl]-3-methylbutyrate is dissolved in 10 ml of methylene chloride. To this solution is added 0.179 ml of triethylamine, and the mixture is stirred at room temperature for 45 minutes. The solvent is distilled off under reduced pressure and the residue is chromatographed on silica gel, elution being carried out with ethyl acetate- hexane (1:1) to give 196 mg of the above-identified compound as a colorless oil.
   IR(Neat): 1752, 1696, 1370, 1138, 1070, 986 cm⁻¹ OH
   NMR(CDC1₃) 6: 1.25s(Bu ), 1.40s & 1.55s(Me₂C-), 2.01s (COCH₃), 2.25s & 2.34s 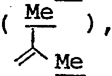 2.50br s, 3.65d(J=5Hz,C₃-H), 5.75 & 5.85ABq(J=5Hz, -OCH₂0), 5.89d(J=5Hz) Rf [AcOEt-hexane (1:1)]: 0.44

### Reference Example 3

### (1-Methyl-lH-tetrazol-5-yl)thioacetyl chloride

1) In 20 ml of water is dissolved 4.00 g of sodium hydroxide, and 11.6 g of 5-mercapto-l-methyl-lH-tetrazole is then dissolved therein. Separately, 9.45 g of monochloroacetic acid is dissolved in 200 ml of water, and 5.30 g of sodium carbonate is added portionwise to make the solution neutral. To this solution is added the above aqueous solution of the sodium salt of 5-mercapto-l-methyl-1H-tetrazole and the mixture is stirred under heating at 80-85°C for 16.5 hours. After cooling, the insoluble matter is filtered off and the filtrate is washed with ethyl acetate (200 mlx2), The aqueous solution is cooled, adjusted to pH 2 with phosphoric acid and extracted with ethyl acetate (100 mlx 3). The ethyl acetate layers are combined, washed with saturated aqueous sodium chloride and dried over magnesium sulfate. The solvent is then distilled off under reduced pressure. To the residue is added ether-hexane (1:1) and the mixture is cooled to give a crystalline precipitate. Hexane is added,and the crystals are recovered by filtration and dried to give 10.17 g of (1-methyl-lH- tetrazol-5-yl)thioacetic acid, melting at 105-107°C.
   IR(Nujol): 1734, 1288, 1204, 1172, 920, 704 cm⁻¹
   NMR(CDCl₃-DMSO-d₆) 6_{:} 3.97s(Me), 4.10s(CH₂)
   Elemental analysis:
   Calcd. for C₄H₆N₄0₂S: C, 27.58; H, 3.47; N, 32.17
   Found : C, 27.42; H, 3.44; N, 32.38
   _{Rf} [AcOEt-AcOH-H20(8:1:1)]: 0.53
2) In 50 ml of methylene chloride is suspended 4.35 g of (l-methyl-lH-tetrazol-5-yl)thioacetic acid and, following addition of 2.35 ml of thionyl chloride, the suspension is stirred under reflux for 3 hours and cooled. The solvent is then distilled off to give a crystalline residue. A 1:1 mixture of ether and hexane is added and the crystals are recovered by filtration, washed with the same mixed solvent and dried to give 3.84 g of the above-identified compound.
   IR(_{N}ujol): 1798, 976, 96₆, ₇₂₄, ₇₀₀ cm⁻¹
   NMR(CDCl₃) 6: 3.99s(Me), 4.65s(CH₂)

### Reference Example 4

### Pivaloyloxymethyl 2-[(3R,4R)-3-(1-hydroxyisopropyl)4-(1-methyl-lH-tetrazol-5-yl)thioacetylthio-2-oxoazetidin-l-yl]-3-methylcrotonate

In 14.2 ml of methylene chloride is dissolved 481 mg of pivaloyloxymethyl 2-[(3R,4R)-4-(benzothiazol-2-ylthio)-3-(1-hydroxyisopropyl)-2-oxoazetidin-1-yl)-3-methylcrotonate, and, under ice-water cooling and stirring, 22.4 ml of 0.1 N NaOH is added. With stirring at the same temperature, 258 mg of (1-methyl-1H-tetrazol-5-yl)thioacetyl chloride and 246 mg of triphenylphosphine are added. With continued stirring at the same temperature, 70 mg portions of (1-methyl-1H-tetrazol-5-yl)thioacetyl chloride are added after 20 and 30 minutes, respectively. The reaction mixture is left standing at room temperature for 45 minutes and then stirred further for 15 minutes. After phase separation, the aqueous layer is extracted with methylene chloride. These methylene chloride layers are combined, washed with water and dried over magnesium sulfate. The solvent is then distilled off under reduced pressure. The orange- colored residue is chromatographed on 10% water-inactivated silica gel and elution is carried out with ethyl acetate- hexane (2:1) to give 139 mg of the above-identified compound as an oil.

IR(KBr): 1748, 1372, 1136, 1074, 1026, 990 cm⁻¹ OH NMR(CDCl₃) 6: 1.19s(Bu^{t}), 1.35s & 1.55s(Me₂C-), 1.99s & 2.24s 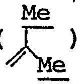 , 2.74br(OH), 3.68d(J=_{5H}z, C₃-H), 4.03s(tetrazole-CH₃), 4.35s(COCH₂S), 5.80 & 5.93ABq(J=6, -OCH₂0) Rf[AcOEt-hexane (2:1)]: 0.35

### Reference Example 5

### 5-p-Nitrobenzyloxycarbonylaminovaleric acid

In 15 ml of acetonitrile is suspended 957 mg of 5-aminovaleric acid and, under ice-water cooling and stirring, 1.63 ml of ethyldiisopropylamine is added dropwise. The mixture is stirred at the same temperature for 5 minutes and, after 1.34 ml of trimethylsilyl chloride is added all at once, it is stirred further for 30 minutes. Then, a solution of 1.761 g of p-nitrobenzyl chlorocarbonate in 5 ml of acetonitrile, and 1.41 ml of ethyldiisopropylamine are added dropwise in that order, and the mixture is stirred for 30 minutes. To this mixture is added 60 ml of ice-water and extraction is carried out with ethyl acetate. The ethyl acetate layer is washed with 1 N HCl and water in that order and extracted with 20 ml of 1 N NaOH, The alkaline aqueous layer is adjusted to pH 2 with phosphoric acid And extracted with ethyl acetate. This ethyl acetate layer is washed with aqueous sodium chloride and dried over magnesium sulfate. The solvent is then distilled off under reduced pressure to give a crystalline residue. Hexane is added and the crystals are recovered by filtration, washed with hexane and ether in that order, and dried to give 2.047 g of the above-identified compound, melting at 100-102°C.

IR(Nujol): 3330, 1692, 1534, 1516, 1345, 1278 cm Elemental analysis: _{C}alcd. for C₁₃H₁₆N₂O₆: C, 52.70; H, 5.44; N, 9.46 Found : C, 52.90; H, 5.57; N, 9.18 Rf[AcOEt-AcOH-H₂O(8:1:1)]: 0.89 _{NMR}(CDC1₃-DMSO-d₆) 6: 1.4-2.0m(-CH₂CH₂-), 2.1-2.6m (CH₂CO), 2.9-3.5m(CH₂NH), 5.20s (COOCH₂), 5.83m(NH), 7.52 & 8.19ABq (J=9Hz, aromatic protons), 10.7br (COOH)

### Reference Example 6

### 5-p-Nitrobenzyloxycarbonvlaminovaleryl chloride

In 10 ml of methylene chloride is suspended 1.48 g of 5-p-nitrobenzyloxycarbonylaminovaleric acid, followed by addition of 0.47 ml of thionyl chloride. The mixture is stirred under reflux for 2 hours and the solvent is distilled off under reduced pressure to give the above-identified compound as a light-yellow oil in quantitative yield.

IR(Neat): 1804, 1796, 1726, 1526, 1350, 1250 cm⁻¹ NMR(CDC1₃) δ: 1.3-2.2m(-CH₂CH₂-), 2.3-4.0m(COCH₂ & CH₂NH), 5.21s(CO₂CH₂), 7.53 & 8.22ABq(J= 9Hz, aromatic protons)

### Reference Example 7

### (3R,4R)-4-Acetylthio-3-(l-hydroxyisopropyl)-2-oxoazetidine

1) In 30 ml of ethyl acetate is dissolved 473 mg of pivaloyloxymethyl 2-[(3R,4R)-4-acetylthio-3-(1-hydroxyisopropyl)-2-oxoazetidin-l-yl]-3-methylcrotpnate, and under cooling at -78°C on a dry ice-acetone bath, ozone is bubbled into the above solution. When the solution has turned blue, TLC is performed to confirm the disappearance of the starting compound. The excess ozone is purged with N₂ gas and the ethyl acetate layer is washed with 3% aqueous solution of sodium hydrogen sulfite (25 mlx2) and aqueous sodium chloride in that order and dried over magnesium sulfate. The solvent is then distilled off under reduced pressure to give 406 mg of a colorless syrupy residue which is crude pivaloyloxymethyl 2-[(3R,4R)-4-acetylthio-3-(1-hydroxyisopropyl)-2-oxoazetidin-1-yl]-2-oxoacetate.
   IR(Neat): 1807, 1760, 1712, 1124, 1102 cm⁻¹
2) In 20 ml of methanol is dissolved 406 mg of the above pivaloyloxymethyl 2-oxoacetate thus obtained, and one drop of 28% solution of sodium methylate in methanol is added using a TLC capillary pipette. The mixture is stirred at room temperature for an hour and the solvent is distilled off under reduced pressure. The residue is dissolved in ethyl acetate, washed with aqueous sodium chloride and dried over magnesium sulfate. The solvent is then distilled off under reduced pressure and the residue is chromatographed on 10% water-inactivated silica gel, elution being carried out with ethyl acetate-hexane (2:1) to give 107 mg of the above-identified compound as a colorless syrupy substance. Upon standing, this product crystallizes. IR(KBr): 3460, 1752, 1728, 1700, 1674, 992 cm⁻¹ NMR(CDCl₃) 6: 1.35s & 1.50s 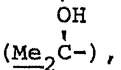 2.38s(COCH₃), 2.61br s(OH), 3.46d(J=5Hz,C₃-H), 5.50d(J=5Hz, C₄-H), 6.73br(NH) Elemental analysis: Calcd. for C₈H₁₃NO₃S; C, 47.27; H, 6.45; N, 6.89 Found ; C, 47.00; H, 6.47; N, 6.92 Rf[AcOEt-hexane (2:1)]: 0.30

### Reference Example 8

### p-Nitrobenzyl 2-[(3R,4R)-4-acetylthio-3-(l-hydroxyisopropyl)-2-oxoazetidin-l-yl]-2-hydroxyacetate

In 20 ml of benzene is suspended 1.00 g of p-nitrobenzyl glyoxalate monohydrate and the suspension is stirred under reflux, with the byproduct water being removed with a Dean-Stark trap. After 1.5 hours, 447 mg of (3R,4R)-4- acetylthio-3-(1-hydroxyisopropyl)-2-oxoazetidine is added and the mixture is stirred under reflux for 5 hours. After cooling, the solvent is distilled off under reduced pressure and the residue is chromatographed on silica gel. Elution with ethyl acetate-chloroform (1:2) gives 748 mg of the above-identified compound as a colorless syrupy substance. This product is a mixture of 2 diastreomers with respect to the 2-position of the hydroxyacetyl moiety.

_{IR}(_{KB}r): 1760, 151₈, ₁₃₄₈, ₁₂₂₀, ₁₁₀₈ cm 1 OH NMR(DMSO-d₆) δ: 1.23s & 1.35s(Me₂C ), 2.28s & 2.36s (each 1.5H,COCH₃), 3.68d(J=5Hz,C₃-H), 4.88s(OH), 5.12d(J=7Hz,0.5H,N-CHOH-), 5.30s& 5.33s(COOCH₂), 5.45d(J=6Hz,0.5H, N-CHOH-), 5.67d & 5.75d(each J=5Hz, C₄-H), 6.7ld(J=6Hz,0.5H,N-CHOH-), 6.75d (J=7Hz,0.5H,N-CHOH-), 7.71d(J=9Hz,2H, aromatic protons), 8.22d & 8.24d(each J=9Hz,each lH, aromatic protons) Rf[AcOEt-CHCl₃(1:2)]: 0.08 & 0.15

### Reference Example 9

### p-Nitrobenzyl 2-[(3R,4-R)-4-acetylthio-3-(1-hydroxyisopropyl)-2-oxoazetidin-1-yl]-2-txiphenylphosphoranylideneacetate

1) In 79.3 ml of dry THF is dissolved 1.635 g of p-nitrobenzyl 2-[(3R,4R)-4-acetylthio-3-(1-hydroxyisopropyl)-2-oxoazetidin-l-yl]-2-hydroxyacetate and the mixture is cooled to -20°C. While stirring the mixture, a solution of 0,919 ml of 2,6-lutidine in 16 ml of dry THF is added dropwise. Then, under cooling at -30 to -20°C, a solution of 0.613 ml of thionyl chloride in 16 ml of dry THF is added dropwise and the whole mixture is maintained at the same temperature for 25 minutes. The resulting white precipitate is removed by gravity filtration and the mother liquor is distilled under reduced pressure to give p-nitrobenzyl 2-[(3R,4R)-4-acetylthio-3-(1-hydroxyisopropyl)-2-oxoazetidin-l-yl]-2-chloroacetate as a colorless to light-yellow syrupy substance. IR(KBr): 3420, 1768, 1696, 15₂₂, ₁₃₅₀, ₁₂₁₈ cm⁻¹ NMR(CDCl₃) δ: 1.35s & 1.48s 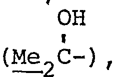 2.33s & 2.38s(each 1.5H,COCH₃), 2.57br(OH), 3.65d(J=6_{Hz}, C₃-H), 5.29s & 5.36s(each 1H,COOCH₂), 5.90d (J=6Hz,C₄-H), 7.17s(CHC1), 7.56 & 8.22ABq (J=9Hz, aromatic protons)
   Rf[AcOEt-hexane (1:1)]: 0.39
2) The above 2-chloroacetate thus obtained is dissolved in 79.3 ml of dry dioxane and, following addition of 0.919 ml of 2,6-lutidine and 2.08 g of triphenylphosphine, the mixture is stirred at room temperature for 6 days. The precipitate is filtered off and the mother liquor is concentrated under reduced pressure. The residue is chromatographed on silica gel and elution is carried out with ethyl acetate-hexane (1:1) and ethyl acetate in that order to give 1.037 g of the above-identified compound as a light-yellow frothy substance.
   IR(KBr): 1752, 1626, 1440, 1348, 1264, 1110, 698 cm⁻¹
   Elemental analysis:
   Calcd. for C₃₅H₃₃N₂O₇SP: C, 64.01; H, 5.07; N, 4.27
   Found : C, 64.68; H, 5.00; N, 4.08
   Rf[AcOEt-hexane (1:1)]: 0.08

### Reference Example 10

### p-Nitrobenzyl (5R, 6R)-6-(1-hydroxyisopropyl)-2-methyl-2- penem-3-carboxylate

In 164 ml of dry toluene is dissolved 328 mg of p-nitrobenzyl 2-[(3R,4R)-4-acetylthio-3-(1-hydroxyisopropyl)-2-oxoazetidin-1-yl]-2-triphenylphosphoranylideneacetate, followed by addition of a small amount of p-hydroquinone. The mixture is stirred in an argon atmosphere at 100°C for 24 hours. After cooling, the solvent is distilled off under reduced pressure and the residue is chromatographed on a silica gel column, elution being carried out with ethyl acetate-hexane (1:1) to give 125 mg of a light-yellow viscous oil. Upon standing, this product crystallizes and, following addition of ether-hexane (1:1), the crystals are recovered by filtration and dried. The procedure gives 74 mg of the above-identified compound as white crystals, melting at 130-132°C.

IR(KBr): 3530, 1760, 1730, 1514, 1346, 1296 cm⁻¹ NMR(CDCl₃) 6: 1.35s & 1.49s 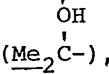 , 2.38s(C₂-CH₃), 2.60br, 4.00d(J=5Hz,C₆-H), 5.13 & 5.47ABq (J=14Hz,COOCH₂), 5.68d(J=5Hz,C₅-H), 7.57 & 8.15ABq(J=9Hz, aromatic protons) Elemental analysis: Calcd. for C₁₇H₁₈N₂0₆S; C, 53.96; H, 4.79; N, 7.40 Found : C, 54.07; H, 4.89; N, 7.45 Rf[AcOEt-CHCl₃(1:4)]: 0.49 Optical rotation: 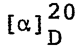 +81.9° (C=1.025%, CHCl₃)

### Reference Example 11

### (5R,6R)-6-(1-Hydroxyisopropyl-)-2-methyl-2-penem-3-carboxylic acid

In 17.9 ml of ethyl acetate is dissolved 169 mg of p-nitrobenzyl (5R,6R)-6-(1-hydroxyisopropyl)-2-methyl-2- penem-3-carboxylate and, following serial addition of 17.9ml of water, 93.8 mg of sodium hydrogen carbonate and 358 mg of 5% palladium-on-carbon, the mixture is stirred in a hydrogen atmosphere for 1 hour and 20 minutes. Water is added and the catalyst is filtered off. After phase separation of the filtrate, the aqueous layer is washed with ethyl acetate and, following addition of 282 mg of citric acid monohydrate, it is extracted with methylene chloride (25 ml x 4) and dried over magnesium sulfate. The solvent is then distilled off under reduced pressure to give a light-yellow syrupy residue. Upon addition of ether and standing, this product crystallizes. There is thus obtained 34 mg of the above-identified compound, m.p. 107-109°C (decompn.).

IR(KBr): 3470, 1760, 1680, 1583, 1286 cm⁻¹ OH NMRCDCl₃) δ: 1.33s & 1.47s(Me₂C-), 2.38s(C₂-CH₃), 3.95d (J=4Hz,C₆-H), 5.65d(J=4Hz,C₅-H)

Elemental analysis: Calcd. for C₁₀H₁₃N0₄S: C, 49.37; H, 5.39; N, 5.76 Found : C, 50.00; H, 5.62; N, 5.13 Rf [AcOEt-AcOH-H₂O(8:1:1)]: 0.72 Optical rotation: 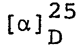 +224° (C=0.605%, CHC1₃) UV 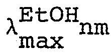 : 264(ε,3470), 312.5(ε,6990)

### Reference Example 12 Pivaloyloxymethyl 2-[(3R,4R)-3-(1-hydroxyisopropyl)-4-(1-methyl-1H-tetrazol-5-yl)thioacetylthio-2-oxoazetidin-1-yl]-2-oxoacetate

In 80 ml of ethyl acetate is dissolved 1.706 g of pivaloyloxymethyl 2-[(3R,4R)-3-(1-hydroxyisopropyl)-4-(1-methyl-1H-tetrazol-5-yl)thioacetylthio-2-oxoazetidin-1-yl]-3-methylcrotonate and the solution is cooled to -78°C in a dry ice-acetone bath. Then, under stirring, ozone is bubbled into the above solution until the reaction mixture turns blue. The excess ozone is purged with N₂ gas and the mixture is washed with 3% aqueous solution of sodium hydrogen sulfite and aqueous sodium chloride in that order and dried over magnesium sulfate. The solvent is then distilled off under reduced pressure to give 1.528 g of the above-identified compound as a frothy substance.

_{IR}(K_{B}r): 1808, 1760. 1716. 13₇4. ₁21₂. ₁₁0₆ cm⁻¹ NMR(CDCl₃) 6: 1.22s(Bu^{t}), 1.36s & 1.53s 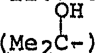 3.25br(OH), 3.85d(J=6Hz,C₃-H), 4.05s(tetrazole-CH₃), 4.44s (COCH₂S), 5.95s(COOCH₂O), 6.20d(J=6Hz,C₄-H)

### Reference Example 13

### (3R,4R)-3-(1-Hydroxyisoropyl)-4-(1-methyl-1H-tetrazol-5-yl)-thioacetylthio-2-oxoazetidine

In 60 ml of methanol is dissolved 1.482 g of pivaloyloxymethyl 2-[(3R,4R)-3-3(1-hydroxyisopropyl)-4-(1-methyl-1H-tetrazol-5-yl)thioacetylthio-2-oxoazetidin-1-yl]-2-oxoacetate, and three drops of 28% solution of sodium methylate in methanol are added using a TLC capillary pipette. The mixture is stirred at room temperature for 7 hours and the solvent is distilled off under reduced pressure. The residue is chromatographed on 10% water-inactivated silica gel and elution is carried out with ethyl acetate to give 472 mg of the above-identified compound as s white frothy substance.

I_{R}(KBr): 1756, 1682, 1374, 1174, 988, 704 cm⁻¹ NMR(CDCl₃) 6: 1.27s & 1.47s 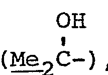 , 3.13br s(OH), 3.65d (J=5Hz,C₃-H), 4.00s(tetrazole-CH ), 4.41s (COCH₂S), 5.55d(J=5Hz,C4-H), 7.23br s(NH) Rf [AcOEt]: 0.44

### Reference Example 14

### p-Nitrobenzyl 2-[(3R,4R)-3-(1-hydroxyisopropyl)-4-(1-methyl-1H-tetrazol-5-yl)thioacetylthio-2-oxoazetidine-1-yl-)-2-hydroxyacetate

In 14.4 ml of benzene is suspended 360 mg of p-nitrobenzyl glyoxalate monohydrate and the suspension is stirred under reflux for an hour, with the water being removed with a Dean-Stark trap. In 6 ml of chloroform is dissolved 500 mg of (3R,4R)-3-(1-hydroxyisopropyl)-4-(1-methyl-1H-tetrazol-5-yl)thioacetylthio-2-oxoazetidine, and the solution is poured into the above-mentioned suspension. The solvent is then distilled off and, following addition of 30 ml of benzene, the mixture is stirred under reflux for 40.5 hours. After cooling, the solvent is distilled off under reduced pressure to give 898 mg of a white frothy residue which is, though crude, the above-identified compound.

IR(KBr): 3430, 1760, 1525, 1352, 1220, 1110 cm⁻¹ OH NMR(DMSO-d₆) δ: 1.16s & 1.34s(Me₂C-), 3.69d(J=5Hz, C₃-H), 3.97s(tetrazole-CH₃), 4.40s & 4.48s (each 1H,COCH₂S), 4.90br s(Me COH), 5,04d(J=6Hz,0,5H,N-CHOH), 5.32br s(COOCH₂), 5.43d(J=6Hz,0.5H,N-CHOH), 5.67d & 5.75d (each J=5Hz,each 0.5H,C₄-H), 6.74d(J=6Hz, N-CHOH), 7.70d(J=9Hz,2H, aromatic protons), 8.23d & 8.25d (each J=9Hz, each 1H, aromatic protons) Rf[AcOEt]: 0.51 & 0.58

### Reference Example 15

### p-Nitrobenzyl 2-[(3R,4R)-3-(1-hydroxyisopropyl)-4-(1-methyl-1H-tetrazol-5-yl)thioacetylthio-2-oxoazetidin-1-yl]-2-triphenylphos^{p}horanylidenjeacetate

1) In 10 ml of dry tetrahydrofuran is dissolved p-nitrobenzyl 2-[(3R,4R)-3-(1-hydroxyisopropyl)-4-(1-methyl-1H-tetrazol-5-yl)thioacetylthio-2-oxoazetidin-1-yl]-2-hydroxyacetate [synthesized from 100 mg of (3R,4R)-3-(1-hydroxyisopropyl)-4-(1-methyl-1H-tetrazol-5-yl)thio- acetylthio-2-oxoazetidine in the same manner as Reference Example 14]. The solution is cooled to -30°C, and under stirring, thereto are added dropwise a solution of 0.0617 ml of 2,6-lutidine in 2 ml of dry tetrahydrofuran and a solution of 0.0419 ml of thionyl chloride in 2 ml of dry tetrahydrofuran in that order. After the completion of addition, the mixture is stirred at -30 to -20°C for 30 minutes. The white precipitate is removed by gravity filtration and the mother liquor is distilled under reduced pressure. The residue is chromatographed on 10% water-inactivated silica gel and elution is carried out with ethyl acetate-hexane (4:1) to give a yellow viscous oil of p-nitrobenzyl 2-[(3R, 4R)-3-(1-hydroxyisopropyl)-4-(1-methyl-1H-tetrazol-5-yl)-thioacetylthio-2-oxoazetidine-1-yl]-2-chloroacetate.

### Rf[AcOEt-hexane(4:1)]: 0.52 & 0.57

2) The above 2-chloroacetate thus obtained is dissolved in 1 ml of dry dimethylformamide, and 0.0183 ml of 2,6-lutidine, 83.2 mg of triphenylphosphine and 26.3 mg of potassium iodide are added in that order. The mixture is stirred in an argon atmosphere at room temperature for 3 hours, poured into water and extracted with ethyl acetate. The ethyl acetate layer is washed with aqueous sodium chloride and dried over magnesium sulfate. The solvent is then distilled off under reduced pressure and the residue is chromatographed on 10% water-inactivated silica gel, elution being carried out with ethyl acetate-hexane (4:1), ethyl acetate and ethyl acetate-ethanol (10:1) in that order to give 43 mg of the above-identified compound as a light-yellow viscous oil.

IR(KBr): 3470, 1754, 1628, 1524, 1444, 1352, 1270, 1112, 700 cm Rf [AcOEt]: 0.15

### Reference Example 16

### Pivaloyloxymethyl 6-(1-hydroxyethyl)-6-isocyanopenicillanate

In 10 ml of dimethylacetamide is dissolved 2.00 g of 6β-isocyanopenicillanic acid and the solution is cooled to -10°C. To this solution is added dropwise 0.362 ml of acetaldehyde and, then, under stirring at -10 to -5°C, 2 ml aqueous solution of 1.0 g of potassium carbonate is added dropwise. The mixture is stirred at the same temperature for 30 minutes and poured into 50 ml of ice water, and the resulting precipitate is dissolved by addition of 20 ml of ethyl acetate. The solution is phase-separated with sodium chloride and the aqueous layer is extracted with ethyl acetate- hexane (1e3) (80 ml x 3). These organic layers are combined, washed with water and dried over magnesium sulfate. The solvent is then distilled off under reduced pressure to give 1.832 g of a brown syrupy residue which is, though crude, the above-identified compound. This product is considered to be a mixture of compounds having absolute configurations involving R and S isomers for both the 6-position and the 1'-position (the carbon atom to which the hydroxy group of the hydroxyethyl side chain is attached).

IR(Neat): 2980, 2130, 1796, 1754, 1244, 1112ₜ 990 cm⁻¹ NMR(CDC13) 6: 1.20s(Bu^{t}), 4.63br s(C3-H), 5.59br s(C₅-H), 5.95m(CO₂CH₂)(Assignable signals only) Rf[AcOEt-hexane(1:2)]: 0.33

### Reference Example 17

### Pivaloyloxymethyl 6β-[(S)-1-hydroxyethyl]penicillanate [referred to as 1¹ (S) isomer] and pivaloyloxymethyl 6β-[(R,S)-1-hydroxyethyl]penicillanate [referred to as l'(R,S) isomer]

In 5.73 ml of toluene is dissolved 580 mg of pivaloyloxymethyl 6-(1-hydroxyethyl)-6-isocyanopenicillanate, followed by addition of 48.3 mg of azobisisobutyronitrile and 0.618 ml of tri-n-butyltin hydride. The mixture is stirred in argon atmosphere at 80-85°C for 15 minutes. The solvent is then distilled off under reduced pressure and the residue is chromatographed on silica gel, elution being carried out with ethyl acetate-hexane (1:1) to give 74 mg of l'(S) isomer and 187 mg of l'(R,S) isomer each as a cololress viscous oil.

IR(KBr): l'(S)isomer;y 2980, 1760, 1158, 1112, 988 cm⁻¹ l'(R,S)isomer; identical to those for l'(S)isomer NMR(CDCl₃) 6: l'(S)isomer; 1.21s(Bu^{t}), 1.39d(J=_{6Hz}, CH₃CHOH), 1.50s & 1.64s(C₂-Me₂), 2.3br(OH), 3.51dd(J=10 & 4Hz,C₆-H), 4.21m(CH₃CHOH), 4.37s(C₃-H), 5.38d(J=4Hz,C₅-H), 5.68 & 5.82ABq(J=SHz,COOCH2) l'(R,S)isomer: [l'(R)isomer only]; 1.21s (Bu^{t}), 1.36d(J=6Hz,CH₃CHOH), 1.48 & 1.62s (C₂-Me₂), ~3.0br(OH), 3.48dd(J=8 & 4Hz, C₆-H), ~4.2m(CH₃CHOH), 4.35s or 4.39s(C₃-H), 5.31d(J=4Hz,C₅-H), 5.68 & 5.82ABq(J=5Hz, COOCH₂) Rf[AcOEt-hexane(1:1): l'(S)isomer 0.51 l'(R)isomer 0,43

### Reference Example 18

### Pivaloyloxymethyl 6β-[(R,S)-1-tert-butyldimethylsilyloxyethyl]penicillanate

In 1 ml of dimethylacetamide is dissolved 187 mg of pivaloyloxymethyl 6β-[(R,S)-1-hydroxyethyl]penicillanate and, following addition of 110 mg of tert-butyldimethylsilyl chloride and 49.6 mg of imidazole, the mixture is stirred at room temperature for 2 hours and allowed to stand overnight. To this mixture is added water and the mixture is extracted with ethyl acetate, washed with aqueous sodium chloride and dried over magnesium sulfate. The solvent is then distilled off under reduced pressure and the residue is chromatographed on silica gel, elution being carried out with ethyl acetatehexane (1:2) to give 165 mg of the above-identified compound as a colorless viscous oil.

IR(KBr): 1778, 1155, 1106, 988, 834 cm⁻¹ NMR(CDC1₃) δ: 0.07s & 0.10s(Me2Si-), 0.85s & 0.89s(Bu^{t} -Si-), 1.19s(Bu^{t}-CO), 1.34d(J=6Hz,CH₃CHOH), 1.50s & 1.65s(C₂-Me₂), 3.54dd(J=10 &̅ 4Hz, C₆-H), ~4.2m(CH₃CHOH), 4.35s & 4.42s(C₃-H), 5.30d & 5.38d(each J=4Hz, C₅-H), 5.74 & 5.87ABq(J=6Hz, C⁰₂^{CH}₂) Rf[AcOEt-hexane(1:2)]: 0.74(for both isomers)

### Reference Example 19

### Pivaloyloxymethyl 6-(1-tert-butyldimethylsilyloxyethyl)-6-isocyanopenicillanate

In 4 ml of dimethylacetamide is dissolved 786 mg of pivaloyloxymethyl 6-(1-hydroxyethyl)-6-isocyanopenicillanate and, following addition of 433 mg of t-butyldimethylsilyl chloride and 195 mg of imidazole, the mixture is stirred at room temperature for 11 days. To the reaction mixture is added water and the mixture is extracted with ethyl acetate. The ethyl acetate layer is washed with aqueous sodium chloride and dried over magnesium sulfate. The solvent is then distilled off under reduced pressure and the residue is chromatographed on a silica gel column, elution being carried with ethyl acetate-hexane (1:2) to give 280 mg of the above-identified compound as a colorless viscous oil.

IR(KBr): 2130, 1796, 1760, 1112, 994, 832 cm⁻¹ NMR(CDC13) 6: 0.13s & 0.15s(Me₂Si-), 0.90s(Bu^{t} -Si-), 1.21s(CO₂CH₂), 1.36d & 1.49d(each J=6Hz, CH₃CHOH), 1.53s & 1.67s(C₂-Me₂), 4.28q(J= 6Hz,CH₃CHOH), 4.61s(C₃-H), 5.49s & 5.51s (C₅-H), 5.87s(CO₂CH₂) Rf[AcOEt-hexane(1:2)]: 0.81(for all the 4 isomers)

### Reference Example 20

### Pivaloyloxymethyl 6β-[(S)-1-tert-butyldimethylsilyloxyethyl]-penicillanate 1-oxide (referred to as Isomer A) and pivaloyloxymethyl 6β-[(R,S)-1-tert-butyldimethylsilyloxyethyl]-penicillnate 1-oxide

In 29.3 ml of methylene chloride is dissolved 4.55 g of pivaloyloxymethyl 6β-[(R,S)-1-tert-butyldimethylsilyloxyethyl]penicillanate and, under ice-water cooling and stirring, a solution of 1.69 g of m-chloroperbenzoic acid in 37 ml of methylene chloride is added dropwise. The mixture is stirred at the same temperature for an hour. The methylene chloride is then distilled off under reduced pressure and the residue is dissolved in 150 ml of ethyl acetate. This solution is washed with saturated aqueous sodium hydrogen carbonate and aqueous sodium chloride in that order and dried over magnesium sulfate. The solvent is distilled off under reduced pressure, and a 1:2 mixture of ethyl acetate and hexane is added to cause crystallization. The crystals are recovered by filtration, washed with hexane and dried to give 575 mg of white crystals. This product does not contain Isomer A but is a mixture of at least two of the other three isomers (referred to as Isomer Group B). The crystallization mother liquor is concentrated and chromatographed on silica gel and elution is carried out with ethyl acetate-hexane (1:2) to give 426 mg of a single isomer, i.e. Isomer A, as white crystlas, with 2.13 g of a mixture of isomers being additionally obtained as a colorless viscous oil. This product, when standing in the cold, solidifies as a whole.

m.p.: Isomer A, 121-123°C; Isomer Group B, 116-117°C IR(KBr): Isomer Group B: 1770, 1756, 1112, 1008, 840 cm⁻¹ Isomer A ; 1788, 1780, 1168, 1104, 998 cm⁻¹ NMR(CDC1₃) 6: Isomer A; O.lls & 0.15s(Me₂Si-), 0.90s (Bu^{t}-Si-), 1.22s(COBu^{t}), 1.22s & 1.65s (C₂-Me₂), 1.25d(J=10Hz,CH₃CHOSi), 3.63dd (J=10 & 5Hz, C₆-H), 4.54s(C₃-H), 4.61d (J=5Hz,C₅-H), ~4.85m(CH₃CHOSi), 5.64 & 5.90ABq(J=5Hz,CO₂CH₂) Elemental analysis: Calcd. for C₂₂H₃₉NO₇ SSi; C, 53.96; H, 8.03; N, 2.86 Found: Isomer A: C, 54.25; H, 8.27; N, 2.80

Isomer Group B: C, 54.27; H, 8.11; N, 2.91 Rf[AcOEt-hexane(1:2)]:
Isomer A : 0.59
Other isomers: 0.39

### Reference Example 21

### Pivaloyloxymethyl 2-[(3R,4R)-4-(benzothiazol-2-yl-dithio)-3-[(R,S)-1-tert-butyldimethylsilyloxyethyl]-2-oxoazetidin-1-yl]-3-methylcrotonate

1) To 78 mg of pivaloyloxymethyl 6β-[(R,S)-1-tert-butyldimethylsilyloxyethyl]penicillanate 1-oxide are added 5 ml of toluene and 26.7 mg of 2-mercaptobenzothiazole and the mixture is stirred under reflux for 3.5 hours, with the byproduct water being removed with a Dean-Stark trap. After cooling, the solvent is distilled off under reduced pressure to give pivaloyloxymethyl 2-[(3R,4R)-4-(benzothiazol-2- yldithio)-3-[(R,S)-1-tert-butyldimethylsilyloxyethyl]-2- oxoazetidin-1-yl]-3-methylbutyrate as a colorless viscous oil in quantitative yield. This product is a mixture of two isomers with respect to the asymmetric carbon atom to which the tert-butyldimethylsilyloxy group is bonded.

Rf(AcOEt-hexane (1:2)];
l'S isomer 0.56
l'R isomer 0.61
2) The above ester thus obtained is dissolved in 5 ml of methylene chloride and, under cooling with ice water, 0.0223 ml of triethylamine is added. The mixture is stirred at the same temperature for an hour and, following addition of 0.17 ml of 1 N HC1, further amounts of water and methylene chloride are added, and the mixture is allowed to stand for phase separation. The methylene chloride layer is washed with water and dried over magnesium sulfate. The solvent is then distilled off under reduced pressure to give 94 mg of the above-identified compound as a colorless viscous oil.

IR(KBr): 1775, 1754, 1432, 1134, 1072, 1006 cm⁻¹ NMR(CDCl₂) 6: 0.12s & 0.17s & 0.22s (Me₂Si-), 0.95s 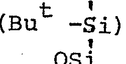 , 1.10s(COBu^{t}), 1.50d(J=6_{H}z, 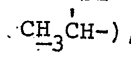 , 2.07s & 2.09s 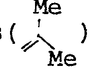 , 3.59dd (J=5 & 2Hz,C₃-H), ~4.45m(CH₃CHOSi), 5.33d (J=5Hz,0.5H,C₄-H), 5.38d(J=5Hz,0.5H, C₄-H), 5.53 & 5.68ABq(J=5Hz,1H,CO₂CH₂O), 5.57 & 5.72ABq(J=5Hz,1H,CO₂CH₂O), 7.1-7.4m (2H,aromatic protons), 7.6-7.9m(2H,aromatic protons)

Rf[AcOEt-hexane (1:2)]:
l'S isomer 0.59
1'R isomer 0.63

### Reference Example 22

### Pivaloyloxymethyl 2-[(3R,4R)-3-[(R,S)-l-tert-butyldimethyl- silloxethl]-4-(1-methl-1H-tetrazol-5-yl)thioacetylthio-2-oxoazetidin-1-yl]-3-methylcrotonate

In 16.6 ml of methylene chloride is dissolved 665 mg of pivaloyloxymethyl 2-[(3R,4R)-4-(benzothiazol-2-yldithio)-3-[(R,S)-1-tert-butyldimethylsilyloxyethyl]-2-oxoazetidin-1-yl]-3-methylcrotonate and, under ice-cooling, 26.1 ml of 0.1 N aqueous sodium hydroxide is added. Then, under stirring at the same temperature, 301 mg of (1-methyl-lH- tetrazol-5-yl)thioacetyl chloride and 287 mg of triphenylphosphine are added and, after 20 and 30 minutes, 82-mg portions of (1-methyl-1H-tetrazol-5-yl)thioacetyl chloride are further added. After 45 minutes, the reaction temperature is returned to room temperature and stirring is continued for 15 minutes. The mixture is allowed to stand for phase separation. The methylene chloride layer is washed with water and dried over magnesium sulfate. The solvent is then distilled off under reduced pressure and the residue is chromatographed on 10% water-inactivated silica gel, elution being carried out with ethyl acetate-hexane (1:2) to give 388 mg of the above-identified compound as a colorless viscous oil.

IR(KBr): 1768, 1750, 1136, 1070, 990 cm⁻¹ NMR(CDC13) δ: 0.06s & 0.12s(Me₂Si-), 0.88s(Bu^{t}-Si-), 1.18s(CO₂CH₂O), 1.26d & 1.40d(each J=6Hz, each 1.5H, CH₃CHOSi), 1.92s & 2.19s 3.61dd(J=3 & 6Hz,C₃-H), 3.94s 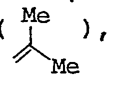 (tetrazole-CH₃), 4.26s(COCH₂S), ~4.3m (CH₃CHOSi), 5.65-5.9m(3H,CO₂CH₂O & C₄-H) Rf[AcOEt-hexane (1:2)]: 0.25 (for both isomers) Reference Example 23

### (3R,4R)-3-[(R,S)-1-tert-butyldimethylsilyloxyethyl]-4-(l-methyl-lH-tetrazol-5-yl)thioacetylthio-2-oxoazetidine

1) In 18.3 ml of ethyl acetate is dissolved 388 mg of pivaloyloxymethyl 2-[(3R,4R)-3-[(R,S)-1-tert-butyldimethylsilyloxyethyl]-4-(1-methyl-1H-tetrazol-5-yl)thio- acetylthio-2-oxoazetidin-l-yl]-3-methylcrotonate and the solution is cooled to -78°C in a dry ice-acetone bath. Then, under stirring, ozone is bubbled into the above solution until the solution turns blue. The reaction mixture is stirred at 0°C for 30 minutes, washed with 3% aqueous sodium hydrogen sulfite (15.2 mlx2) and aqueous sodium chloride in that order and dried over magnesium sulfate. The solvent is then distilled off under reduced pressure to give 361 mg of a colorless viscous oil which is crude pivaloyloxymethyl 2-[(3R,4R)-3-[(R,S)-1-tert-butyldimethylsilyloxyethyl]-4-(1-methyl-1H-tetrazol-5-yl)thioacetylthio-2-oxoazetidin-1-yl]-2-oxoacetate.

IR(KBr); 1816, 1764, 1714, 1372_{f} 1106, 968 cm⁻¹ NMR(CDC1₃) δ: 0.05s & 0.11s(Me₂Si-), 0.89s(Bu^{t}-Si-), 1.22s(Bu^{t}CO), 1.51d(J=7Hz,CH₃CHSi), 3.6-3.8m(C₃-H), 3.97s(tetrazole -CH₃), 4.37s (COCH₂S), ~4.4m(CH₃CHSi), 5.81s(CO₂CH₂O), 6.00d & 6.10d(each J=6Hz,each 0.5H,C₄-H)

2) The above ester thus obtained is dissolved in 12.2 ml of methanol and one drop of 28% solution of sodium methylate in methanol is added using a TLC capillary pipette. The mixture is stirred at room temperature for 6 hours and allowed to stand overnight. The methanol is distilled off under reduced pressure and the residue is dissolved in ethyl acetate, washed with aqueous sodium chloride and dried over magnesium sulfate. The solvent is then distilled off under reduced pressure and the residue is chromatographed on 10% water-inactivated silica gel, elution being carried out with ethyl acetate-hexane (1:1) to give 179 mg of the above-identified compound as a colorless viscous oil.

IR(KBr): 1770, 1684, 1258, 966, 840, 784 cm⁻¹ NMR(CDC1₃) δ: 0.10s(Me₂Si-), 0.87s(Bu^{t}-Si-), 1.25d & 1.35d(each J=6Hz, each 1.SH,CH₃CHOSi), ~3.5m(C₃-H), 3.97s(tetrazole-CH₃), ~4.15m (_{CH3CHOS}i), 4.33s(COCH₂S), 5.44d(J=5Hz, C₄-H), 6.88br s & 7.02br s(each 0.5H,NH) Rf[AcOEt-hexane (1:1)]: 0.22(for both isomers)

### Reference Example 24

### p-Nitrobenzyl 2-[(3R,4R)-3-(R,S)-1-tert-butyldimethyl- silyloxyethl]-4-(1-methl-1H-tetrazol-5-yl)thioacetylthio-2-oxoazetidin-1-yl]-2-triphenylphosphoranylideneacetate

1) In 40.8 ml of benzene is suspended 98.1 mg of p-nitroybenzyl glyoxalate monohydrate and the suspension is stirred under reflux for an hour, with the water being removed with a Dean-Stark trap. After cooling, a solution of 179 mg of (3R,4R)-3-[(R,S)-1-tert-butyldimethylsilyloxyethyl]-4-(1-methyl-1H-tetrazol-5-yl)thioacetylthio-2- oxoazetidine in 3 ml of 1,2-dichloroethane is added to the above suspension. Refluxing with stirring for 22 hours does not cause the reaction to proceed to a considerable extent. Therefore, the solvent is distilled off under reduced pressure. To the residue is added 10 ml of toluene and the mixture is stirred under reflux for 8 hours. The solvent is then distilled off under reduced pressure to give a yellow viscous oil which is crude p-nitrobenzyl 2-[(3R,4R)-3-[(R,S)-1-tert-butyldimethylsilyloxyethyl]-4-(1-methyl-1H-tetrazol-5-yl)thioacetylthio-2-oxoazetidin-1-yl]-2-hydroxyacetate.

2) The above ester thus obtained is dissolved in 13.6 ml of dry tetrahydrofuran and the solution is cooled to -30°C. Then, under stirring, a solution of 0.0841 ml of 2,6-lutidine in 2.5 ml of dry tetrahydrofuran and a solution of 0.0572 ml of thionyl chloride in 2.5 ml of dry tetrahydrofuran are added dropwise in that order and the mixture is kept at -30 to -20°C for 30 minutes. The white precipitate is filtered off and the filtrate is concentrated under reduced pressure to give a light-yellow viscous oil which is crude p-nitrobenzyl 2-[(3R,4R)-3-[(R,S)-l-tert-butyldimethylsilyloxyethyl]-4-(l-methyl-lH-tetrazol-5-yl)thio- acetylthio-2-oxoazetidin-l-yl]-2-chloroacetate.

3) The above ester thus obtained is dissolved in 2.73 ml of dimethylformamide and, following serial addition of 0.0499 ml of 2,6-lutidine, 227 mg of triphenylphosphine and 71.7 mg of potassium iodide, the mixture is stirred at room temperature for 3 hours and 15 minutes. The mixture is poured into water and extracted with ethyl acetate. The ethyl acetate layer is washed with aqueous sodium chloride and dried over magnesium sulfate. The solvent is then distilled off under reduced pressure and the residue is chromatographed on 10% water-inactivated silica gel, elution being carried out with ethyl acetate-hexane(2:l) to give 196mg of the above-identified compound as a yellow viscous oil.

Rf[AcOEt-hexane (2:1)]: 0.49 (for both isomers)

### Reference Example 25

### Pivaloyloxymethyl 6β-[(S)-1-tert-butyldimethylsilyloxyethyl]-penicillanate

Using 493 mg of pivaloyloxymethyl 6S-[(S)-1-hydroxyethyl]penicillanate, the procedure of Reference Example 18 is repeated to give 300 mg of the above-identified compound as a colorless viscous oil. This product crystallizes upon standing, m.p. 85-88°C.

IR(KBr): 1778, 1772(sh), 1760, 1108, 990, 836 cm⁻¹ NMR(CDCl₃) 6: 0,08s(Me₂Si-), 0.83s(Bu^{t}-Si), 1.18s(COBu^{t}), 1.35d(J=6Hz;CH₃CHOSi), 1.49s & 1.63s (C₂-Me₂), 3.53dd(J=10 & 4Hz,C₆-H), ~4.3m (CH₃CHOSi), 4.33s(C3-H), 5.35d(J=4Hz, C₅-H), 5.72 & 5.85ABq(J=6Hz,CO₂CH₂) Elemental analysis: Calcd. for C₂₂H₃₉NO₆SSi: C, 55.78; H, 8.30; N, 2.96 Found ; C, 55.89; H, 8.53; N, 2.97

### Reference Example 26

### Pivaloyloxymethyl 6β-[(S)-1-tert-butyldimethylsilyloxyethyl]-penicillanate 1-oxide (Isomer A, Isomer C)

In 5.42 ml of methylene chloride is dissolved 298 mg of pivaloyloxymethyl 6β-[(S)-1-tert-butyldimethylsilyloxyethyl]penicillanate and, under ice-water cooling and stirring, 110.5 mg of m-chloroperbenzoic acid is added. The mixture is stirred at the same temperature for an hour and the solvent is distilled off under reduced pressure. The residue is dissolved in ethyl acetate, washed with aqueous sodium hydrogen carbonate and aqueous sodium chloride in that order and dried over magnesium sulfate. The solvent is then distilled off under reduced pressure and the residue is chromatographed on silica gel, elution being carried out with ethyl acetate-hexane (1:2) to give 77 mg of Isomer A and 90 mg of Isomer C each as white crystals. IR, NMR, TLC and mp of Isomer A are in complete agreement with those of Isomer A obtained in Reference Example 20. Isomer C is an isomer to Isomer A due to the asymmetry of the sulfoxide moiety and has the following characteristics:

mp 91-94°C IR(KBr): 1786, 1764, 1104, 1080, 984 cm⁻¹ NMR(CDC1₃) 6: 0.12s(Me2Si-), 0.85s(Bu^{t}-Si), 1.22s(Bu^{t}CO), 1.29s & 1.58s(C2-Me2), 1.40d(J=6Hz, CH₃CHOSi), 3.72dd(J=7 & 5Hz), 4,20s(C₃-H), 4.49d(J=5Hz,C₅-H), 5.67 & 5.88ABq(J=6Hz, CO₂CH₂) Elemental analysis: Calcd. for C₂₂H₃₉NO₇SSi: C, 53.96; H, 8.03; N, 2.86 Found : C, 53.86; H, 8.27; N, 2.63

### Reference Example 27

### Pivaloyloxymethyl 2-[(3R,4R)-4-(benzothiazol-2-yl-dithio)-3-[(S)-1-tert-butyldimethylsilyloxyethyl]-2-oxoazetidin-1-yl]-3-methylcrotonate

Using 487 mg of pivaloyloxymethyl 6β-[(S)-1-tert-butyldimethylsilyloxyethyl]penicillanate 1-oxide (Isomer A obtained in Reference Example 26), the procedure of Reference Example 21 is repeated to give 630 mg of the above-identified compound as a light-yellow syrupy substance.

IR(KBr): 1774, 1750(sh), 1430, 1132, 1072, 1020, 1006 cm⁻¹ NMR(CDC1 ) 6: 0.13s & 0.17s(Me₂Si-), 0.96s(Bu^{t}-Si), 1.09s(Bu^{t}CO), 1.50d(J=6Hz,CH₂CHOSi), 2.06s 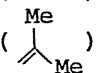 , 3.63dd(J=2 & 5Hz,C₃-H), ~4.5m(CH₃CHOSi), 5.40d(J=5Hz,C₄-H), 5.59 & 5.73ABq(J=5Hz,CO₂CH₂), 7.0-7.5m & 7.5-8.0m (each 2H, aromatic protons)

### Reference Example 28

### Pivaloyloxymethyl 2-[(3R,4R)-3-[-(S-)-1-tert-butyldimethylsilyloxyethyl]-4-(1-methyl-1H-tetrazol-5-yl)thioacetylthio-2-oxoazetidin-1-yl)-3-methylcrotonate

Using 630 mg of pivaloyloxymethyl 2-[(3R,4R)-4-(benzothiazol-2-yldithio)-3-[(S)-1-tert-butyldimethylsilyloxyethyl]-2-oxoazetidin-1-yl]-3-methylcrotonate, the procedure of Reference Example 22 is repeated to give 395 mg of the above-identified compound as a colorelss viscous oil.

IR(KBr): 1770, 1746(sh), 1138, 1066, 990 cm⁻¹ NMR(CDCl₃) δ : 0.06s & 0.11s(Me₂Si-), 0.88s(Bu^{t}-Si), 1.18s (Bu^{t}-CO), 1.26d(J=6_{H}z,CH_{3CH}OSi), 1.91s & 2.19s( 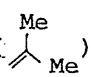 , 3.62dd(J=3 & 6Hz,C₃-H), 3.94s(tetrazole-CH₃), 4.26s(COCH₂S), ~4.4m(CH₃CHOSi), 5.74d(J=6Hz,C₄-H), 5.78s (CO₂CH₂)

### Reference Example 29

### (3R,4R)-3-[(S)-1-tert-butyldimethylsilyloxyethyl]-4-(1-methyl-1H-tetrazol-5-yl)tioacetylthio-2-oxoazetidine

1) Using 395 mg of pivaloyloxymethyl 2-[(3R,4R)-3-[(S)-1-tert-butyldimethylsilyloxyethyl]-4-(1-methyl-1H-tetrazol-5-yl)thioacetylthio-2-oxoazetidin-1-yl]-3-methylcrotonate, the procedure 1) of Reference Example 23 is repeated to give 371 mg of pivaloyloxymethyl 2-[(3R,4R)-3-[(S)-1-tert-butyldimethylsilyloxyethyl]-4-(lemethyl-1H-tetrazol-5-yl)thioacetylthio-2-oxoazetidin-1-yl]-2-oxoacetate as a colorless viscous oil.

IR(KBr): 1816, 1760, 1715, 1370, 1105, 968 cm⁻¹ NMR(CDC1₃) 6: 0.04s & 0.08s(Me₂Si-), 0,87s(Bu^{t}-Si), 1.20s(Bu^{t}-CO), 1.25d(J=6Hz,CH₃CHOSi), 3.84dd(J=2 & 6Hz,C₃-H), 4.37s(COCH₂S), ~4.45m(CH₃CHOSi), 5.80s(CO₂CH₂), 6.09d (J=6Hz,C₄-^{H})

2) Using the above ester thus obtained, the procedure 2) of Reference Example 23 is repeated to give 188 mg of the above-identified compound as a colorless viscous oil. IR(KBr): 1765, 1684, 1260, 964, 840, 784 cm⁻¹ NMR(CDC1₃) δ: 0.08s(Me₂Si-), 0.84s(Bu^{t}-Si-), 1.24d (J=6Hz,CH₃CHOSi), 3.53dd(J=5 & 5Hz,C₃-H), 3,96s(tetrazole-CH₃),~4.15m(GH₃CHOSi), 4.33s(COCH₂S), 5.44d(J=5Hz,C₄-H), 6.98br s(NH)

### Reference Example 30

### p-Nitrobenzyl 2-[(3R,4R)-3-[(S)-1-tert-butyldimethylsilyloxyethyl]-4-(1-methyl-1H-tetrazol-5-yl]thioacetylthio-2- oxoazetidin-1-yl]-2-triphenylphosphoranylideneacetate

Using 188 mg of (3R,4R)-3-[(S)-1-tert-butyldimethylsilyloxyethyl]-4-(1-methyl-1H-tetrazol-5-yl)thioacetylthio-2-oxoazetidine, the procedure of Reference Example 24 is repeated to give 192 mg of the above-identified compound as a yellow viscous oil.

### Reference Example 31

### Pivaloyloxymethyl 2-[(3R, 4R) -3-[(R,S)-1-tert-butyldimethylsilyloxyethyl]-4-(5-p-nitrobanzyloxycarbonylaminovaleroyl)-thio-2-oxoazetidin-1-yl]-3-methylcrotonate

In 30.6 ml of methylene chloride is dissolved 1.207 g of pivaloyloxymethyl 2-[(3R,4R)-4-(benzothioazol-2-yl-dithio)-3-[(R,S)-l-tert-butyldimethylsilyloxyethyl]-2-oxoazetidin- l-yl]-3-methylcrotonate and the solution is cooled with ice water. To this solution is added dropwise 48.1 ml of 0.1 N aqueous sodium hydroxide and, under stirring at the same temperature, 529 mg of triphenylphosphine is added, after which a 15-ml portion of a solution of 1.42 g 5-p-nitro- benzyloxycarbonylaminovaleryl chloride in 23.1 ml of methylene chloride is added all at once. After 20 and 30 minutes, 4-ml portions of the above solution of 5-p-nitrobenzyloxy- carbonylaminovaleryl chloride are added, and the mixture is allowed to stand at room temperature for 45 minutes and then stirred at that temperature for 15 minutes. The methylene chloride layer is separated, washed with aqueous sodium chloride and dried over magnesium sulfate. The solvent is themdistilled off under reduced pressure and the residue is chromatographed on silica gel, elution being carried out with ethyl acetate-hexane (1:1) to give 776 mg of the above-identified compound as a colorless viscous oil.

IR(KBr): 1754, 1728, 1524, 1348, 1072 cm⁻¹ NMR(CDC1₃) δ: 0.09s & 0.14s & 0,16s(Me₂si-), 0.91s(Bu^{t-}Si-), 1.21s(Bu^{t}-CO), 1.42d(J=7Hz,CH₃CHOSi), ~1.6m(-CH₂CH₂-), 1.98s & 2.18s 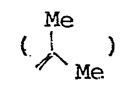 , ~2.55m(CH₂COS), ~3.15m(CH₂NHCO₂), 3.61dd(J=4 & 6Hz,C₃-H), ~4.25m(CH₃CHOSi), 5.16s(NHCO₂CH₂), 5.78d & 5.81d(each J=6Hz, C₄-H), 5.83s(OCH₂0), 7.41 & 8,09ABq(J=9Hz, aromatic protons) Rf[AcOEt-hexane (1:1)]: 0.62(for both isomers)

### Reference Example 32

### (3R, 4R)-3-[(R,S)-1-tert-butyldimethylsilyloxyethyl]-4-(5-p-nitrobenzyloxycarbonylaminovaleroyl)thio-2-oxoazetidine

1) In 30.5 ml of ethyl acetate is dissolved 776 mg of pivaloyloxymethyl 2-[(3R,4R)-3-[(R,S)-1-tert-butyldimethylsilyloxyethyl]-4-(5-p-nitrobenzyloxycarbonylamino- valeroyl)thio -2-oxoazetidin-1-yl] -3-methylcrotonate and the solution is cooled at -78°C on a dry ice-acetone bath. Ozone is bubbled into the above solution until the reaction mixture turns blue. The mixture is stirred at 0°C for 30 minutes, washed with 3% aqueous sodium hydrogen sulfite and aqueous sodium chloride in that order and dried over magnesium sulfate. The solvent is then distilled off under reduced pressure to give 700 mg of pivaloyloxymethyl 2-[(3R,4R)-3-[(R,S)-1-tert-butyldimethylsilyloxyethyl]-4-(5-p-nitro- benzyloxycarbonylaminovaleroyl)thio-2-oxoazetidin-1-yl]-2- oxoacetate as a colorless syrupy substance.

IR(KBr): 1818, 1760, 1716, 1526, 1348, 1108 cₘ⁻¹ NMR(CDCl₃) 6: 0.07s & 0.12s & 0.18s(Me₂Si-), 0.90s (Bu^{t}-Si-), 1.22s(Bu^{t}-CO), 1.40d(J=_{6H}z, CH₃CHOSi), ~1.7m(-CH₂CH₂-), ~2.65m(CH₂COS), ~3.2m(CH₂NHCO₂), ~3.75m(C₃-H), ~4.25m _{(CH3CHOSi)f} 5.16s(NHC02CH2-), 5.84s(OCH₂0), ⁶_{.}^{02d} & 6.14d(each J=6Hz,C₄-H), 7.41 & 8.09ABq(J=9Hz, aromatic protons)

2) The above ester thus obtained is dissolved in 19.5 ml of methanol and two drops of 28% solution of sodium methylate in methanol are added dropwise using a TLC capillary pipette. The mixture is stirred at room temperature for 18.5 hours. The solvent is distilled off under reduced pressure and the residue is dissolved in ethyl acetate. The solution is washed with aqueous sodium chloride and dried over magnesium sulfate. The solvent is then distilled off under reduced pressure and the residue is chromatographed on 10% water-inactivated silica gel, elution being carried out with ethyl acetate-hexane (1:1) to give 311 mg of the above-identified compound as a colorless viscous oil.

IR(K_{B}r): 1765, 1710, 1522, 1346, ₁₂₅₂, ₈₄₀ cm⁻¹ NMR(CDCl₃) 6: O.lls & 0.16s(Me₂Si-), 0.90s(But-Si-), 1.26d & 1.36d(each J=6Hz,CH₃CHOSi), ~1.65m (-CH₂CH₂-), ~2.6m(SCOCH₂), ~3.2m(CH₂NHCO₂), ~3.55m(C₃-H), ~4.15m(CH₃CHOSi), 5.16s (CH₂CH₂), 5.45d(J=5Hz,C₄-H), ~5.7m(NHCO₂), 6.91br s & 7.03br s(each 0.5H,NH), 7.41 & 8.08ABq(J=8Hz,aromatic protons) Rf[AcOEt-hexane (1:1)]: 0.30 (for both isomers)

### Reference Example 33

### p-Nitrobenzyl 2-[(3R,4R)-3-[(R,S)-1-tert-butyldimethylsilyloxyethyl]-4-(5-p-nitrobenzyloxycarbonylaminovaleroyl)thio-2- oxoazetidin-1-yl]-2-triphenylphosphoranylideneacetate

1) In 40 ml of benzene is suspended 131 mg of p-nitrobenzyl glyoxalate monohydrate and the suspension is stirred under reflux for an hour. After cooling, the suspension is poured into 311 mg of (3R,4R)-3-[(R,S)-1-tert-butyldimethylsilyloxyethyl]-4-(5-p-nitrobenzyloxycarbonylamino- valeroyl)thio-2-oxoazetidine for dissolution. The solvent is distilled off under reduced pressure. To the residue is added 13.4 ml of dry toluene and the mixture is stirred under reflux for 12 hours. The solvent is then distilled off under reduced pressure to give a light-yellow syrupy residue which is crude pivaloyloxymethyl-2-[(3R,4R)-3-[(R,S)-1-tert-butyldimethylsilyloxyethyl]-4-(5-p-nitxobenzyloxy- carbonylaminovaleroyl)thio-2-oxoazetidin-1-yl]-2-hydroxyacetate.

Rf [AcOEt-hexane (2:.1)]: 0.53 & 0.46

2) The above ester thus obtained is dissolved in 18.2 ml of dry tetrahydrofuran and the solution is cooled to -30°C. Then, under stirring at -30 to -20°C, a solution of 0.112 ml of 2,6-lutidine in 2.5 ml of dry tetrahydrofuran and a solution of 0.0764 ml of thionyl chloride in 2.5 ml of dry tetrahydrofuran are added dropwise in that order and the mixture is further stirred at the same temperature for 30 minutes. The white precipitate is filtered off and the filtrate is concentrated under reduced pressure to give a light-yellow syrupy residue which is crude pivaloyloxymethyl 2-[(3R,4R)-3-[(R,S)-1-tert-butyldimethylsilyloxyethyl]-4-(5-p-nitrobenzyloxycarbonylaminovaleroyl)thio-2-oxoazetidin- l-yl]-2-chloroacetate.

Rf [AcOEt-hexane (2:1)]: 0,74 (for both isomers)

3) The above ester thus obtained is dissolved in 3.65 ml of dimethylformamide and, following serial addition of 0.0667 ml of 2,6-lutidine, 304 mg of triphenylphosphine and 95.8 mg of potassium iodide, the mixture is stirred at room temperature for 3 hours and 15 minutes. To this mixture is added ice water and the whole mixture is extracted with ethyl acetate. The ethyl acetate layer is washed with aqueous sodium chloride and dried over magnesium sulfate. The solvent is then distilled off under reduced pressure and the residue is chromatographed on 10% water-inactivated silica gel, elution being carried out with ethyl acetate- hexane (2:1) to give 303 mg of the above-identified compound as a light-yellow viscous oil.

Rf [AcOEt-hexane (2:1)]: 0.54 (for both isomers)

### Example 1

### p-Nitrobenzyl (5R,6R)-6-(1-hydroxyisopropyl)-2-(1-methyl-1H-tetrazol-5-yl)thiomethyl)thiomethyl-2-penem-3-carboxylate

In a mixture of 18,3 ml of dry toluene and 10 ml of dry dioxane is dissolved 43 mg of p-nitrobenzyl 2-[(3R,4R)-3-(1-hydroxyiopropyl)-4-(1-methyl-1H-tetrazol-5-yl)thio- acetylthio-2-oxoazetidin-1-yl]-2-triphenylphosphoranylideine- acetate, followed by addition of a trace amount of 1,4-hydroquinone. The mixture is stirred in an argon atmosphere at 100°C for 2 hours and 10 minutes. After cooling, the solvent is distilled off under reduced pressure and the residue is chromatographed on 10% water-inactivated silica gel. Elution with ethyl acetate-hexane (4:1) gives 21 mg of the above-identified compound as a yellow viscous oil.

_{IR}(_{KB}r): 3420, 1780, 1714, 1520, 1348, 1₃₃2, ₁284 cm⁻¹ NMR(CDC1₃) δ: 1.34s & 1.50s 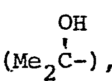 2.54br s(OH), 4.0ls (tetrazolerCH₃), 4.08d(J=5Hz,C₆-H), 4.60 & 4.97ABq(J=15Hz,-CH₂S-), 5.30 & 5.61ABq (J=14Hz,COOCH₂), 5.82d(J=5Hz,C₅-H), 7.78 & 8.39ABq(J=9Hz,aromatic protons) Rf(AcOEt-hexane (4:1)]: 0.51

### Example 2

### Sodium (5R,6R)-6-(l-hydroxyisopropyl)-2-(l-methyl-lH- tetrazol-5-yl)thiomethyl-2-penem-3-carboxylate

In 2 ml of ethyl acetate is dissolved 20 mg of p-nitrobenzyl (5R,6R)-6-(1-hydroxyisopropyl)-2-(1-methyl-1H-tetrazol-5-yl)thiomethyl-2-penem-3-carboxylate and, following serial addition of 2 ml of water, 4 mg of sodium hydrogen carbonate and 40 mg of 5% palladium-on-carbon, the mixture is stirred in a hydrogen atmosphere at room temperature for an hour. The catalyst is then filtered off and the filtrate is washed with ethyl acetate. The aqueous layer is concentrated to about 1 ml under reduced pressure and the concentrate is chromatographed on Sephadex-LH 20 resin, using water as the developer solvent. The active fractions are pooled and lyophilized to give 4.7 mg of the above-identified compound as a white powder.

IR(KBr): 3430, 1764, 1612, 1580(sh), 1380 cm⁻¹ OH _{NMR}(_{D2}0) 6: 1.46s & 1.51s(Me₂C-), 4.22s(tetrazole -CH₃), 4.23d(J=4Hz,C₆-H), 4.53 & 4.86ABq(J=14Hz, CH₂S), 5,86d(J=4Hz,C₅-H) Rf [AcOEt-AcOH-H₂0 (8:1;1)]: 0.57 Optical rotation: 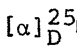 +30.7° (C=0.61%,H₂O) UV 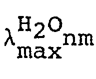 : 256(ε,4070), 315(ε,3490)

### Example 3 .

### p-Nitrobenzyl (5R,6R)-6-[(S)-1-tert-butyldimethylsilyloxyethyl]-2-(1-methyl-1H-tetrazol-5-yl)thiomethyl-2-penem-3-carboxylate (referred to as l'S isomer) and p-nitrobenzyl (5R,6R)-6-[(R)-1-tert-butyldimethylsilyloxyethy1]-2-(1-methyl-lH-tetrazol-5-yl)thiomethyl-2-penem-3-carboxylate (referred to as 1'R isomer)

In 50 ml of dry toluene is dissolved 196 mg of p-nitrobenzyl 2-[(3R,4R)-3-[(R,S)-1-tert-butyldimethylsilyloxyethyl]-4-(l-methyl-lH-tetrazol-5-yl)thioacetylthio-2- oxoazetidin-1-yl]-2-triphenylphosphoranylideneacetate and, following addition of a trace amount of p-hydroquinone, the mixture is stirred in an argon atmosphere at 100°C for 2 hours. The solvent is then distilled off under reduced pressure and the residue is chromatographed on 10% water-inactivated silica gel, elution being carried out with ethyl acetate-hexane (1:1) to give the above-identified compound (1'S isomer, 33 mg; 1'R isomer, 29 mg) as a light-yellow viscous oil. l'S isomer contains a trace amount of p-nitrobenzyl (5R,6R)-6-[(S)-l-tert-butyldimethylsilyloxyethyl]-2-(1-methyl-1H-tetrazol-5-yl)thiomethylene- penam-3-carboxylate and 1'R isomer contains a tract amount of p-nitrobenzyl (5R,6R)-6-[(R)-1-tert-butyldimethylsilyloxyethyl]-2-(1-methyl-lH-tetrazol-5-yl)thiomethylenepenam-3-carboxylate.

IR(KBr): l'S isomer; 1790, 1526, 1350, 1320, 838 cm⁻¹ 1'R isomer; 1796, 1525, 1352, 1320_{f} 840 cm⁻¹ NMR(CDCl₃) δ: l'S isomer 0,08s & 0.10s(Me₂Si-), 0.86s Bu^{t}-Si-), 1.41d(J=6Hz,CH₃CHOSi), 3.87dd (J=10 & 4Hz,C₆-H), 3.96s(tetrazole-CH₃), ~4.3m(CH₃CHOSi), 4.53 & 4.93ABq(J=14Hz, C₂-CH₂), 5.21 & 5.47ABq(J=14Hz,CO₂CH₂), 5.67d(J=4Hz,C₅-H), 7.62 & 8.23ABq(J=9_{H}z, aromatic protons) 1'R isomer 0.09s & 0.13s(Me₂Si-), 0.92s (Bu^{t}-Si-), 1.19d(J=6Hz,CH₃CHOSi), 3.93dd (J=10 & 4Hz,C₆-H), 3.96s(tetrazole-CH₃), ~4.3m(CH₂CHOSi), 4.49 & 4.95ABq(J=14_{H}z, C₂-CH₂), 5.25 & 5.43ABq(J=14Hz,CO₂CH₂), 5.58d(J=4Hz,C₅-H), 7.62 & 8.22ABq(J=9Hz, aromatic protons)

Rf [AcOEt-hexane (1;1)]: l'S isomer 0.60 1'R isomer 0.48

### Example 4

### p-Nitrobenzyl (5R, 6R)-6-[(S)-1-hydroxyethyl]-2-(1-methyl-1H-tetrazol-5-yl)thiomethyl-2-penem-3-carboxylate

In 1 ml of tetrahydrofuran is dissolved 46 mg of p-nitrobenzyl (5R,6R)-6-[(S)-l-tert-butyldimethylsilyloxyethyl]-2-(1-methyl-1H-tetrazol-5-yl)thiomethyl-2-penem-3-carboxylate and, following addition of 0.0445 ml of acetic acid and a solution of 50.8 mg of tetra-n-butylammonium fluoride in 1 ml of tetrahydrofuran, the mixture is stirred at room temperature for 26 hours. The solvent is distilled off under reduced pressure and the residue is dissolved in ethyl acetate, washed with water, aqueous sodium hydrogen carbonate and aqueous sodium chloride in that order and dried over magnesium sulfate. The solvent is then distilled off under reduced pressure and the residue is chromatographed on 10% water-inactivated silica gel, elution being carried out with ethyl acetate-hexane (4:1) to give 25 mg of the above-identified compound as a colorless viscous oil.

This product contains a trace amount of p-nitrobenzyl (5R,6R)-6-[(S)-1-hydroxyethyl]-2-(1-methyl-1H-tetrazol-5-yl)thiomethylenepenam-3-carboxylate.

IR(KBr): 1780, 1522, 1350, 1320, 1275 cm⁻¹ NMR(CDC1₃) 6: 1.47d(J=6Hz,CH₃CHOH), 2.40br(OH), 3.87dd (J=10 & 4Hz,C₆-H), 3.98s(tetrazole-CH₃), 4.3m(CH₃CHOH), 4.53 & 4.87ABq(J=14Hz,C₂-CH₂), 5.21 & 5.46ABq(J=14Hz,CO₂CH₂), 5.7ld(J=4Hz, C₆-H), 7.62 & 8.22ABq(J=9Hz,aromatic protons) Rf [AcOEt-hexane (4 :1)]: 0.47

### Example 5

### p-Nitrobenzyl (5R,6R)-6-[(R)-1-hydroxyethyl]-2-(1-methyl-1H-tetrazol-5-yl)thiomethyl-2-penem-3-carboxylate

In 0.63 ml of tetrahydrofuran is dissolved 29 mg of p-nitrobenzyl (5R,6R)-6-[(R)-1-tert-butyldimethylsilyloxyethyl]-2-(l-methyl-lH-tetrazol-5-yl)thiomethyl-2-penem-3-carboxylate and, following addition of 0.0281 ml of acetic acid and a solution of 32 mg of tetra-n-butylammonium fluoride in 0.63 ml of tetrahydrofuran, the mixture is stirred at room temperature for 85 hours. The solvent is distilled off under reduced pressure and the residue is dissolved in ethyl acetate, washed with water, aqueous sodium hydrogen carbonate and aqueous sodium chloride in that order and dried over magnesium sulfate. The solvent is then distilled off under reduced pressure and the residue is chromatographed on 10% water-inactivated silica gel, elution being carried out with ethyl acetate-hexane (4:1) to give 12 mg of the above-identified compound as a colorless viscous oil.

This product contains a trace amount of p-nitrobenzyl (5R,6R)-6-[(R)-1-hydroxyethyl]-2-(1-methyl-1H-tetrazol-5-yl)thiomethylenepenam-3-carboxylate.

IR(KBr): 1780, 1636, 1524, 1350, 1320 cm⁻¹ NMR(CDC1₃) 6: 1.25d(J=6Hz,CH₃CHOH), 2.6br(OH), 3.82dd (_{J}=8 & 4Hz), 3.95s(tetrazole-CH₃), 4.3m (CH₃CHOH), 4.54 & 4.86ABq(J=15Hz,C₂-CH₂), 5,21 & 5.46ABq(J=14Hz,CO₂CH₂), 5.65d(J=4Hz, C₅-H), 7.61 & 8.22ABq(J=9Hz,aromatic protons) Rf [AcOEt-hexane (4:1)]: 0,44

### Example 6

### p-Nitrobenzyl (5R,GR)-6-[-(S)-1-hydroxyethyl]-2-(1-methyl-1H-tetrazol-5-yl)thiomethylenepenam-3-carboxylate (referred to as exo isomer) and p-nitrobenzyl (5R,6R)-6-[(S)-1-hydroxyethyl]-2-(1-methyl-1H-tetrazol-5-yl)thiomethyl-2-penem-3-carboxylate (referred to as endo isomer)

Using 954 mg of crude p-nitrobenzyl (5R,6R)-6-[(S)-1-tert-butyldimethylsilyloxyethyl]-2-(1-methyl-1H-tetrazol-5-yl)thiomethyl-2-penem-3-carboxylate, the procedure of Example 4 is repeated and the resulting product is chromatographed on 10% water-inactivated silica gel. The viscous oil that is obtained is chromatographed further on silica gel and elution is carried out with ethyl acetate-hexane (4:1) to give 37 mg of the exo isomer as a light-yellow viscous oil and 410 mg of the endo isomer as a colorless viscous oil. IR, NMR and TLC of the endo isomer are in agreement with those of the product obtained in Example 4.

IR(KBr): exo isomer 1786, 1756, 1648, 1525, 1350 cm⁻¹ NMR(CDC1₃) δ: exo isomer 1.41d(J=6Hz), CH₃CHOH), 3.74dd (J=10 & 4Hz,C6-H), 3.98s(tetrazole-CH₃), ~4.3m(CH₃CHOH), 5.32s(CO₂CH₂), 5.5ld(J=lHz, C₃-H), 5.65d(J=4Hz,C₋-H), 6.75d(J=lHz, 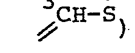 , 7.56 & 8.20ABq(J=8Hz,aromatic protons) Rf [AcOEt-hexane (4:1)]: 0.56

### Example 7

### Sodium (5R,6R)-6-[(S)-1-hydroxyethyl]-2-(1-methyl-1H-tetrazol-5-yl)thiomethyl-2-penem-3-carboxylate

In 18 ml of ethyl acetate is dissolved 187 mg of p-nitrobenzyl (5R, 6R)-6-[(S)-1-hydroxyethyl]-2-(1-methyl-1H-tetrazol-5-yl)thiomethyl-2-penem-3-carboxylate and 18 ml of water, 35 mg of sodium hydrogen carbonate and 384 mg of 5% palladium-on-carbon are added in that order. The mixture is stirred in a hydrogen atmosphere at room temperature for an hour. The catalyst is then filtered off and the filtrate is washed with ethyl acetate and concentrated to about 2 ml under reduced pressure. The concentrate is chromatographed on Sephadex LH-20 resin, using water as the developer solvent. The active fractions are pooled and lyophilized to give 61 mg of the above-identified compound as a white powder.

IR(KBr): 1768, 1606, 1380, 1302, 730 cm⁻¹ NMR(D₂0) δ: 1.40d(J=6Hz,CH₃CHOH), 3.91dd(J=10 & 4Hz, C₆-H), 4.12s(tetrazole-CH₃), ~4.23m(CH₃CHOH), 4.49 & 4.71ABq(J=14Hz,C₂-CH₂), 5.71d(J=4Hz, C₅-H) Rf [AcOEt-AcOH-H₂0 (8:1:1)]: 0.47 Optical rotation: 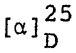 + 110.9° (C=0.67%, H₂O) UV: 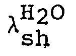 259nm(ε,4290), 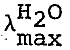 309nm(ε,4620)

### Example 8

### Sodium (5R,6R)-6-[(S)-1-hydroxyethyl]-2-(1-methyl-1H-tetrazol-5-yl)thiomethylenepenam-3-carboxylate

In 4 ml of ethyl acetate is dissolved 37 mg of p-nitrobenzyl (5R,6R)-6-[(S)-1-hydroxyethyl]-2-(1-methyl-1H-tetrazol-5-yl)thiomethylenepenam-3-carboxylate and, following serial addition of 4 ml of water, 7 mg of sodium hydrogen carbonate and 74 mg of 5% palladium-on-carbon, the mixture is stirred in a hydrogen atmosphere at room temperature for an hour. The catalyst is filtered off and the filtrate is washed with ethyl acetate and concentrated to about 1 ml under reduced pressure. The concentrate is chromatographed on Sephadex LH-20 resin, using water as the developer solvent. The active fractions are pooled and lyophilized to give 15 mg of the above-identified compound as a white powder.

IR(KBr): 1768, 1620, 1380, 1325, 1176, 714 cm-1 NMR(D₂0) 6: 1.34d(J=6Hz,CH₃CHOH), 3.84dd(J=10 & 4Hz, C₆-H), 4.07s(tetrazole-CH₃), ~4.15m(CH₃CHOH), 5.35d(J=lHz,C₃-H), 5.64d(J=4Hz,C₅-H), 6.50d (J=1Hz, =CH-s) Rf [AcOEt: AcOH:H₂O (8:1;1)]: 0.30 Optical rotation: 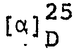 +106.1° (C=0.605%,H₂O) UV: 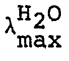 262nm (ε,9680)

### Example 9

### Sodium (5R,6R)-6-[(R)-1-hydroxyethyl]-2-(1-methyl-1H-tetrazol-5-yl)thiomethyl-2-penem-3-carboxylate (referred to as endo isomer) and sodium (5R,6R)-6-[(R)-1-hydroxyethyl]-2-(1-methyl-1H-tetrazol-5-yl)thiomethylenepenam-3-carboxylate (referred to as exo isomer)

In 4.67 ml of ethyl acetate is dissolved 49 mg of p-nitrobenzyl (5R,6R)-6-[(R)-1-hydroxyethyl]-2-(1-methyl- l_{H}-tetrazol-5-yl)thiomethyl-2-penem-3-carboxylate and, following serial addition of 4.67 ml of water, 9 mg of sodium hydrogen carbonate and 98 mg of 5% palladium-on-carbon, the mixture is stirred in a hydrogen atmosphere at room temperature for an hour. The catalyst is then filtered off and the filtrate is washed with ethyl acetate and concentrated to about 1 ml under reduced pressure. The concentrate is chromatographed on Sephadex LH-20 resin, using water as the developer solvent. The active fractions are pooled and lyophilized to give 7.5 mg of the endo isomer and 4.5 mg of the exo isomer each as a white powder.

IR(KBr): endo isomer; 1770, 1620, 1384, 1515, 705 cm⁻¹ exo isomer ; 1768, 1624, 1388, 1178, 706 cm⁻¹ NMR(D₂O) δ: endo isomer 1.23d(J=6Hz,CH₃CHOH), 3.90dd (J=9 & 4H_{Z},C₆-H), 4.14s(tetrazole-CH₃), 5.70d(J=4_{H}z) exo isomer 1.26d(J=6Hz,CH₃CHOH), ~3.9m (C₆-H), 4.14s(tetrazole-CH₃),5.41br s(C₃-H), 5.63d(J=4Hz,C₅-H), 6.53br s(=CH-S) Rf [AcOEt-AcOH-H₂0 (8:1:1)]: endo isomer; 0.24 exo isomer ; 0.18 UV: endo isomer H20 260nm(ε,4240), 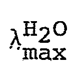 308nm(ε,4140) exo isomer 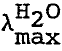 260nm(ε,6850)

### Example 10

### p-Nitrobenzyl (5R,6R)-6-[(S)-1-tert-butyldimethylsilyloxyethyl]-2-(1-methyl-1H-tetrazol-5-yl)thiomethyl-2-penem-3-carboxylate

Using 192 mg of p-nitrobenzyl 2-[(3R,4R)-3-[(S)-1-tert-butyldimethylsilyloxyethyl]-4-(1-methyl-1H-tetrazol-5-yl)thioacetylthio-2-oxoazetidin-l-yl]-2-triphenylphos- phoranylidene acetate, the procedure of Example 3 is repeated to give 46 mg of the above-identified compound as a light-yellow viscous oil. IR, NMR and TLC of this product are in complete agreement with those of the 1'S isomer obtained in Exmaple 3.

### Example 11

### p-Nitrobenzyl (5R,6R)-6-[(S)-1-tert-butyldimethylsilyloxyethyl]-2-(4-p-nitrobenzyloxycarbonylaminobutyl)-2-penem-3-carboxylate (referred to as 1'S isomer) and p-nitrobenzyl (5R,6R)-6-[(R)-1-tert-butyldimethylsilyloxyethyl]-2-(4-p-nitrobenzyloxycarbonylaminobutyl)-2-penem-3-carboxylate (referred to as 1'R isomer)

In 70 ml of dry toluene is dissolved 303 mg of p-nitrobenzyl 2-[(3R,4R)-3-[(R,S)-1-tert-butyldimethylsilyloxyethyl]-4-(5-p-nitrobenzyloxycarbonylaminovaleroyl)thio-2-oxoazetidin-1-yl]-2-triphenylphosphoranylideneacetate and, following addition of a trace amount of p-hydroquinone, the mixture is stirred in an argon atmosphere at 100°C for 1 hour and 10 minutes and, then, at 110°C for 6.5 hours. After cooling, the solvent is distilled off under reduced pressure and the residue is chromatographed on 10% water-inactivated silica gel, elution being carried out with ethyl acetate-hexane(1:1) to give 54 mg of the l'S isomer and 93 mg of the 1'R isomer each as a colorelss viscous oil.

IR(KBr) : l'S isomer; 1788, 1717, 1524, 1350, 1318, 12₆0, 840 cm⁻¹ 1'R isomer; 1788, 1716, 1526, 1348, 1316, 1263, 8₃8 cm⁻¹ NMR(CDC1₃) δ: l'S isomer 0.12s(Me₂Si-), 0.89s(Bu^{t}-Si-), 1.43d(J=6Hz,CH₃CHOSi), ~1.62m(-CH₂CH₂-), ~2.9m(C₂-CH₂), ~3.24m(CH₂NHCO₂), 3.86dd (J=10 & 4Hz,C₆-H), ~4.35m(CH₃CHOSi), ~4,9br (NHCO₂), 5.18 & 5.45ABq(J=13Hz,C₃-CO₂CH₂), 5.20s(NHCO₂CH2), 5.64d(J=4Hz,C₅-H), 7.50 & 8.2l,ABq(J=BHz,4H,aromatic protons), 7.60 & 8,21ABq(J=8Hz,4H,aromatic protons) 1'R isomer 0.10s & 0.14s(Me₂Si-), 0.94s (Bu^{t}-Si-),1.23d(J=6Hz,CH₃CHOSi), ~1.61m (-CH₂CH₂-), ~2.9m(C₂-CH₂),~3.23m(CH₂NHCO₂), 3,93dd(J=9 & 4H_{Z},C₆-H), ~4.35m(CH₃CHOSi), ~4.9br(NHCO₂), 5.20s(NHCO₂CH₂), 5.22 & 5.42ABq(J=14Hz,C₃-CO₂CH2), 5.55d(J=4Hz, C₅-H), 7.50 & 8.21ABq(J=8Hz,4H, aromatic protons), 7.60 & 8.21ABq(J=8_{H}z,4_{H},ₐromₐtic protons) Rf [AcOEt-hexane (1:1)]: l'S isomer 0.61 1'R isomer 0.53

### Example 12

### p-Nitrobenzyl (5R,6R)-6-[(S)-1-hydroxyethyl]-2-(4-p-nibrobenzyloxycarbonylaminobutyl)-2-penem-3-carboxylate

In 1 ml of tetrahydrofuran is dissolved 54 mg of p-nitrobenzyl (5R,6R)-6-[(S)-1-tert-butyldimethylsilyl]-oxyethyl]-2-(4-p-nitrobenzyloxycarbonylaminobutyl)-2- penem-3-carboxylate and, following serial addition of 0.0432 ml of acetic acid and a solution of 59.5 mg of tetra-n-butylammonium fluoride trihydrate in 1 ml of tetrahydrofuran, the mixture is stirred at room temperature-for 28.5 hours. The solvent is then distilled off under reduced pressure and the residue is dissolved in ethyl acetate, washed with water, aqueous sodium hydrogen carbonate and aqueous sodium chloride in that order, and dried over magnesium sulfate. The solvent is distilled off under reduced pressure and the residue is chromatographed on 10% water-inactivated silica gel, elution being carried out with ethyl acetate-hexane (2:1) to give 34 mg of the above-identified compound as a colorless viscous oil,

IR(KBr): 1784, 1718, 1520, 1350, 1320 cm⁻¹ NMR(CDCl₃) 6: 1.48d(J=6Hz,CH₃CHOH), ~1.6m(-CH₂CH₂-), 2.04br(OH), ~2.9m(C₂-CH₂), ~3.23m(CH₂NHCO₂), 3,84dd(J=11 & 4Hz,C₆-H), ~4.3m(CH₃CHOH), 4.98br(NHCO₂), 5.18 & 5.45ABq(J=14Hz, C₃-CO₂CH₂), 5.20s(NHCO₂CH)₂, 5.68d(J=4_{Hz}, C₅-H), 7.50 & 8,20ABq(J=8Hz,aromatic protons), 7.61 & 8.21ABq(J=9Hz,aromatic protons) Rf (AcOEt-hexane (2:1)]: 0.31

### Example 13

### p-Nitrobenzyl (5R,6R)-6-[(R)-1-hydroxyethyl]-2-(4-p-nitrobenzyloxycarbonylaminobutyl)-2-penem-3-carboxylate

In 1.5 ml of tetrahydrofuran is dissolved 93 mg of p-nitrobenzyl (5R,6R)-6-[(S)-1-tert-butyldimethylsilyloxyethyl]-2-(4-p-nitrobenzyloxycarbonylaminobutyl)-2-penem-3-carboxylate and, following serial addition of 0.0743 ml of acetic acid and a solution of 102.6 mg of tetra-n-butylammonium fluoride trihydrate in 1.5 ml of tetrahydrofuran, the mixture is stirred at room temperature for 52.5 hours. The solvent is then distilled off under reduced pressure and the residue is dissolved in ethyl acetate, washed with water, aqueous sodium hydrogen carbonate and aqueous sodium chloride in that order, and dried over magnesium sulfate. The solvent is distilled off under reduced pressure and the residue is chromatographed on 10% water-inactivated silica gel, elution being carried out with ethyl acetate-hexane(2:1) to give 46 mg of the above-identified compound as a colorless viscous oil.

IR(KBr): 1780, 1715, 1520, 1348, 1316 cm⁻¹ NMR(CDCl₃) 6: 1.23d(J=6Hz,CH₃CHOH), ~1.6m(-CH₂CH₂-), 2.03br(OH), ~2.85m(C₂-CH₂), ~3.2m(CH₂NHCO₂), 3.80dd(J=9 & 4Hz,C₆-H), ~4.3m(CH₃CHOH), 4.98br(NHCO₂), 5.20s(NHCO₂CH₂), 5.21 & 5.47ABq(J=14Hz,C₃-CO₂CH₂), 5.62d(J=4Hz, C₅-H), 7.52 & 8.25ABq(J=9Hz,4H,aromatic protons), 7.63 & 8.25ABq(J=9Hz,4H, aromatic protons) Rf [AcOEt-hexane (2:1)]: 0.23

### Example 14

250 g of the product of Example 7 was aseptically packed in portions of 250 mg into sterilized dry vials of 12 ml capacity which were vacuum-sealed at 50 mmHg. The contents are dissolved upon addition of 3 ml of distilled water.

## Claims

1. A (6R)-substituted-(5R)-penem-3-carboxylic acid derivative having the formula: 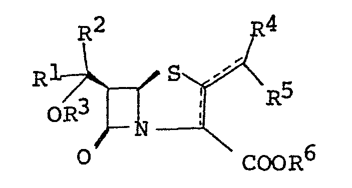 wherein R¹ and R² are the same or different and each is a hydrogen atom or a hydrocarbon group, or R¹ and R ² taken together represent an alkylene group; R is a hydrogen atom or an acyl group; R⁴ is a hydrogen atom, R⁵ is an alkyl, alkylthio, heterocycle-thio, alkoxy or heterocycle-oxy group which may optionally be substituted, or _{R}⁴ and R⁵ taken together represent a lower alkylene group which may optionally be substituted; and R⁶ is a hydrogen atom or an ester residue, or a pharmacologically acceptable salt thereof.

2. A compound as claimed in claim 1, wherein R¹ and _{R}² are the same or different and each is a hydrogen atom or a lower alkyl group, or R¹ and R² taken together represent a lower alkylene group.

3. A compound as claimed in claim 1, wherein R³ is a hydrogen atom. ,

4. A compound as claimed in claim 1, wherein R⁴ is a hydrogen atom and R⁵ is a heterocycle-thio group which may be substituted with a lower alkyl, carboxy-lower alkyl or di-lower alkylamino-lower alkyl group.

5. A compound as climed in claim 1, which is (5R, 6R)-6-[(S)-1-hydroxyethyl)-2-(1-methyl-lH-tetrazol-5-yl)thiomethyl-2-penem-3-carboxylic acid.

6. A compound as claimed in claim 1, which is (5R, 6R)-6-[(S)-1-hydroxyethyl]-2-(1-methyl-1H-tetrazol-5-yl)thio- methylenepenam-3-carboxylic acid.

7. A compound as claimed in claim 1, which is (5R, 6R)-6-[(R)-1-hydroxyethyl]-2-(1-methyl-1H-tetrazol-5-yl)thiomethyl-2-penem-3-carboxylic acid.

8. A compound as claimed in claim 1, which is (5R, 6R)-6-[(R)-1-hydroxyethyl]-2-(1-methyl-1H-tetrazol-5-yl)thiomethylene- penam-3-carboxylic acid.

9. A method of producing a (6R)-substituted-(5R)-penem-3-carboxylic acid derivative of the formula: 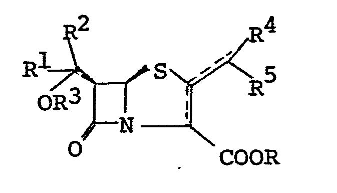 wherein R and R² are the same or different and each is a hydrogen atom or a hydrocarbon group or R¹ and R² taken, together represent an alkylene group; R³ is a hydrogen atom or an acyl group; R⁴ is a hydrogen atom, R⁵ is an alkyl, alkylthio, heterocycle-thio, alkoxy or heterocycle-oxy group which may optionally be substituted, or R⁴ and _{R}⁵ taken together represent a lower alkylene group which may optionally be substituted; R is an ester residue, or a salt thereof, which comprises cyclizing a compound of the formula; 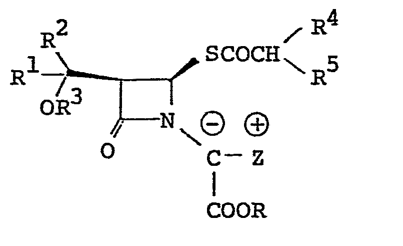 wherein _{R}¹, _{R}², _{R}³, _{R}⁴, R⁵ and R have the same meanings as defined above, and Z is a phosphonio or phosphono group, or a salt thereof.

10. An antimicrobial composition which contains as an active ingredient an effective dose of a (6R)-substituted-(5R)-penem -3-carboxylic acid derivative having the formula: 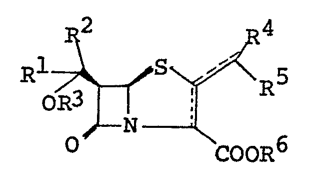 wherein _{R}¹ and R² are the same or different and each is a hydrogen atom or a hydrocarbon group, or R1 and R² taken together represent an alkylene group; R³ is a hydrogen atom or an acyl group; R⁴ is a hydrogen atom, R⁵ is an alkyl, alkylthio, heterocycle-thio, alkoxy or heterocycle-oxy group which may optionally be substituted, or R⁴ and _{R}⁵ taken together represent a lower alkylene group which may optionally be substituted; and R⁶ is a hydrogen atom or an ester residue, or a pharmacologically acceptable salt thereof, together with a suitable carrier or carriers.
